# EUROPÄISCHE PATENTSCHRIFT

(11) **EP 1 038 891 B1**
(45) Veröffentlichungstag und Bekanntmachung des Hinweises auf die Patenterteilung: **22.01.2003**
(21) Anmeldenummer: 00106470.8
(22) Anmeldetag: 24.03.2000
(51) Int. Cl.: C08F 220/00, C08F 220/04, C08G 18/08, A61K 7/06

(54) **Wasserlösliche oder wasserdispergierbare polymere Salze**
Water soluble or water dispersible polymeric salts
Sels de polymères solubles ou dispersibles dans l'eau

(30) Priorität: 26.03.1999 DE 19913875
(43) Veröffentlichungstag der Anmeldung: 27.09.2000
(73) Patentinhaber: BASF AKTIENGESELLSCHAFT, 67056 Ludwigshafen (DE)
(72) Erfinder: Nguyen Kim, Son, 69502 Hemsbach (DE); Sanner, Axel, 67227 Frankenthal (DE); Hössel, Peter, 67105 Schifferstadt (DE)
(74) Vertreter: Kinzebach, Werner, Dr.

(56) Entgegenhaltungen:
- EP-A- 0 619 111
- EP-A- 0 773 246
- US-A- 4 874 604

## Beschreibung

Die vorliegende Erfindung betrifft wasserlösliche oder wasserdispergierbare polymere Salze, die Verwendung dieser Salze sowie kosmetische Mittel, die diese Salze enthalten.

In der Kosmetik werden Polymere mit filmbildenden Eigenschaften zur Festigung, Strukturverbesserung und Formgebung der Haare verwendet. Diese Haarbehandlungsmittel enthalten im Allgemeinen eine Lösung des Filmbildners in einem Alkohol oder einem Gemisch aus Alkohol und Wasser.

Haarfestigungsmittel werden im Allgemeinen in Form von wässrig-alkoholischen Lösungen auf die Haare aufgesprüht. Nach dem Verdampfen des Lösungsmittels werden die Haare an den gegenseitigen Berührungspunkten vom zurückbleibenden Polymer in der gewünschten Form gehalten. Die Polymere sollen einerseits so hydrophil sein, dass sie aus dem Haar ausgewaschen werden können, andererseits aber sollen sie hydrophob sein, damit die mit den Polymeren behandelten Haare auch bei hoher Luftfeuchtigkeit ihre Form behalten und nicht miteinander verkleben. Um eine möglichst effiziente Haarfestigerwirkung zu erzielen, ist es außerdem wünschenswert, Polymere einzusetzen, welche ein relativ hohes Molekulargewicht und eine relativ hohe Glastemperatur (mindestens 10 °C) besitzen.

Ein weiterer aktueller Anspruch an Haarbehandlungsmittel ist es, dem Haar Flexibilität, ein natürliches Aussehen und Glanz zu verleihen, z. B. auch dann, wenn es sich um von Natur aus besonders kräftiges und/oder dunkles Haar handelt.

Bei der Formulierung von Haarfestigern ist außerdem zu berücksichtigen, dass aufgrund der Umweltbestimmungen zur Kontrolle der Emission flüchtiger organischer Verbindungen (VOC = volatile organic compounds) in die Atmosphäre eine Verringerung des Alkoholund Treibmittelgehalts erforderlich ist.

Es ist bekannt, wasserlösliche oder dispergierbare Polyurethane in der Kosmetik einzusetzen. So eignen sie sich z. B. aufgrund ihrer filmbildenden Eigenschaften und einer im Allgemeinen niedrigen Viskosität in Wasser/Ethanol für den Einsatz in der Haarkosmetik, wie z. B. zur Formulierung von Haarsprays.

Die DE-A-42 25 045 und die WO 94/03515 beschreiben die Verwendung von wasserlöslichen oder in Wasser dispergierbaren, anionischen Polyurethanen als Haarfestiger. Diese Polyurethane sind aufgebaut aus
a) mindestens einer Verbindung, die zwei oder mehrere aktive Wasserstoffatome pro Molekül enthält,
b) mindestens einem Säure- oder Salzgruppen enthaltenden Diol und
c) mindestens einem Diisocyanat.

Die in diesen Polyurethanen enthaltenen Säuregruppen können durch Neutralisation mit wenigstens einer Base in die entsprechenden Salze überführt werden. Dazu werden niedermolekulare Amine, wie 2-Amino-2-methylpropanol, Diethylaminopropylamin und Triisopropanolamin eingesetzt.

Die EP-A-619 111 beschreibt die Verwendung von Polyurethanen auf Basis von organischen Diisocyanaten, Diolen und 2,2-Hydroxymethyl-substituierten Carboxylaten in Haarfixierungsmitteln. Zumindest ein Teil der Carbonsäuregruppen wird dabei mit einer organischen oder anorganischen Base, ausgewählt unter Natriumhydroxid, Kaliumhydroxid, 2-Amino-2-methylpropanol, Histidin, Tris(hydroxymethyl)aminomethan und Triethanolamin, neutralisiert.

Die DE-A-195 41 658 beschreibt wasserlösliche bzw. wasserdispergierbare Pfropfpolymere aus einem Polyurethanpräpolymer mit endständigen Isocyanatgruppen und einem freie Aminogruppen enthaltenden Protein.

Die EP-A-636 361 beschreibt eine kosmetische Zusammensetzung, welche in einem kosmetisch verträglichen Träger mindestens einen Pseudolatex auf Basis eines Polykondensates umfasst, das mindestens eine Polysiloxaneinheit und mindestens eine Polyurethanund/oder Polyharnstoffeinheit mit anionischen oder kationischen Gruppen umfasst. Als Neutralisierungsmittel werden dabei Mineralbasen, niedermolekulare Amine und Aminoalkohole, Mineralsäuren und niedermolekulare Carbonsäuren eingesetzt. Die WO 97/25021 hat einen vergleichbaren Offenbarungsgehalt. Die Auswaschbarkeit dieser Filmbildner ist nicht zufriedenstellend. Zudem besitzen sie aufgrund eines hohen Siloxananteils auch nicht die für ein Haarpolymer erforderliche Festigungswirkung.

Die DE-A-195 41 329 und die WO 97/17052 beschreiben Haarbehandlungsmittel, enthaltend ein in Wasser oder in einem Wasser/Alkohol-Gemisch lösliches oder dispergierbares Haarfestigerpolymer und zusätzlich ein in Wasser lösliches oder dispergierbares siloxanhaltiges Salz. Haarspray-Formulierungen auf Basis dieser siloxanhaltigen Salze, einem nicht siloxanhaltigen Haarfestigerpolymer und einem Siliconöl führen zu Filmen, die leicht, z. B. durch mechanische Belastung, von der Haaroberfläche abgelöst werden. Die Festigungswirkung dieser Formulierungen ist daher verbesserungswürdig.

Die DE-A-195 41 326 und die WO 97/17386 beschreiben wasserlösliche oder wasserdispergierbare Polyurethane mit endständigen Säuregruppen, ihre Herstellung und ihre Verwendung. Dabei wird ein in Wasser lösliches oder dispergierbares Polyurethanpräpolymer mit endständigen Isocyanatgruppen mit einer Aminosulfonsäure oder Aminocarbonsäure, insbesondere Taurin, Asparaginsäure und Glutaminsäure, umgesetzt.

Die DE-A-197 09 277 betrifft polysiloxanhaltige Haarfestigungsmittel, enthaltend 0,5 bis 15 Gew.-% carboxylgruppenhaltige Polymere, die in neutralisierter Form wasserlöslich oder wasserdispergierbar sind. Als Neutralisierungsmittel werden dabei Alkalicarbonate, Ammoniak sowie Amine und Aminoalkohole mit maximal 3 Kohlenstoffatomen in der längsten Kohlenstoffkette eingesetzt.

Keines der zuvor genannten Dokumente beschreibt polymere Salze auf Basis von Polymeren mit freien ionogenen Gruppen und Neutralisationsmitteln mit wenigstens zwei dazu komplementären ionogenen Gruppen. Die zuvor beschriebenen Polyurethane führen zu Filmen, die in Bezug auf ihre Flexibilität und somit in Bezug auf die dem Haar verliehene Geschmeidigkeit noch verbesserungswürdig sind.

Es ist bekannt, Copolymere auf der Basis α,β-ethylenisch ungesättigter Mono- und/oder Dicarbonsäuren in Haarpflegemitteln einzusetzen.

Die GB-A-1 321 836 beschreibt Haarfestiger auf der Basis von Copolymeren, die eine ungesättigte Dicarbonsäure und ein Vinyloder Vinylidenmonomer einpolymerisiert enthalten. Dabei sind 5 bis 20 % der Carboxylgruppen mit primären C₄- bis C₁₆-Aminen neutralisiert. Die dabei resultierenden Filme sind weich und klebrig, ihre Festigungswirkung ist verbesserungswürdig.

Die DE-A-29 17 504 beschreibt ein Aerosol-Haarspray auf der Basis eines Copolymers aus mindestens einer ungesättigten Monocarbonsäure und mindestens einem Vinyl- oder Vinylidenmonomer. Dabei sind mindestens 7 bis 100 % der Carboxylgruppen neutralisiert, davon zur Erzielung einer guten Treibgasverträglichkeit mindestens die Hälfte mit einem langkettigen primären, sekundären und/oder tertiären Amin mit 8 bis 20 Kohlenstoffatomen in der längsten Kette. Auch dabei resultieren weiche und klebrige Filme mit verbesserungswürdiger Festigungswirkung.

Die WO 89/12438 beschreibt einen Haarfestiger auf Basis eines Haarpolymers mit Carboxylgruppen, die zu mindestens 40 Mol-% mit einem langkettigen Amin, das ausgewählt ist unter Amidoaminen, N-ethoxylierten Aminen und Etheraminen, neutralisiert sind.

Die zuvor genannten Polyacrylate mit Carbonsäuregruppen, die mit Fettaminen oder ethoxylierten Fettaminen neutralisiert sind, führen zu weichen, klebrigen Filmen mit einer drastisch reduzierten Festigungswirkung. Diese Polymere eignen sich daher nur sehr eingeschränkt für einen Einsatz als Haarfestiger.

Die JP-A-7127480 beschreibt ein Haarbehandlungsmittel auf Basis einer Aminsalz-Lösung eines Copolymers, das eine ungesättigte Carbonsäure einpolymerisiert enthält.

Die JP-A-03206023 beschreibt ein Polymerharz für Haarbehandlungsmittel, welches a) 6 bis 35 Gew.-% Acrylsäure, Methacrylsäure, Itaconsäure oder eine Mischung davon, b) 15 bis 50 Gew.-% wenigstens eines C₁₀- bis C₁₈-Alkyl(meth)- acrylats, c) 15 bis 50 Gew.-% wenigstens eines C₄- bis C₈-Alkyl(meth)- acrylats und d) 0 bis 25 Gew.-% wenigstens eines weiteren hydrophoben Vinylmonomers einpolymerisiert enthält. Die erhaltenen Copolymere werden mit einer Base, ausgewählt unter Ammoniak, Morpholin, Isopropanolamin und Aminoethylpropandiol, neutralisiert.

Die JP-A-03206024 beschreibt ein der JP-A-03206023 vergleichbares Haarfestigerpolymer, das zusätzlich 5 bis 50 Gew.-% eines N-Alkyl-substituierten Acrylamids einpolymerisiert enthält. Die in diesen beiden Dokumenten beschriebenen Haarfestigerpolymere weisen einen hohen Anteil an hydrophoben Monomeren auf. Ihre Auswaschbarkeit ist daher verbesserungswürdig.

Die DE-A-39 01 325 und die DE-A-42 14 305 beschreiben Haarfestigungsmittel, die als Filmbildner ein Copolymerisat auf Basis von tert.-Butyl(meth)acrylat und (Meth)acrylsäure enthalten, wobei die Carboxylgruppen der Copolymerisate teilweise oder vollständig durch Amine neutralisiert sind. Dabei sind die Amine ausgewählt unter Mono-, Di- oder Trialkanolaminen, Alkandiolaminen oder primären, sekundären oder tertiären Alkylaminen. Filme auf Basis dieser Polyacrylate sind im Allgemeinen hart und unflexibel.

Die DE-A-197 09 277 beschreibt polysiloxanhaltige Haarfestigungsmittel auf Basis von Carboxylgruppen-haltigen Polymeren, Polysiloxanen mit primären, sekundären oder tertiären Aminogruppen sowie niedermolekularen Neutralisierungsmitteln. Der Einsatz von nicht weiter funktionalisierten Siloxandiaminen in Haarfestigungsmitteln führt zu Produkten, die leicht, z. B. durch mechanische Belastung, von der Haaroberfläche abgelöst werden. Die Auswaschbarkeit dieser Formulierungen ist zudem verbesserungswürdig.

Keines der zuvor genannten Dokumente beschreibt Polyacrylate mit anionischen Gruppen, die eine kationische Komponente auf Basis eines di- oder polyfunktionellen Amins aufweisen. Die zuvor beschriebenen Polyacrylate führen zu Filmen, die in Bezug auf ihre Flexibilität und somit in Bezug auf die dem Haar verliehene Geschmeidigkeit noch verbesserungswürdig sind.

Der vorliegenden Erfindung liegt die Aufgabe zugrunde, wasserlösliche oder wasserdispergierbare polymere Salze zur Verfügung zu stellen. Diese sollen sich als kosmetisches Mittel, beziehungsweise für einen Einsatz in kosmetischen Mitteln, insbesondere Haarbehandlungsmittel(n), eignen. Vorzugsweise sollen sie klebfreie Filme mit guten flexiblen Eigenschaften bilden, so dass auf ihnen basierende Haarbehandlungsmittel dem Haar Elastizität verleihen.

Überraschenderweise wurde nun gefunden, dass diese Aufgabe durch wasserlösliche oder wasserdispergierbare polymere Salze aus wenigstens einem Polymer mit freien ionogenen Gruppen und einem Neutralisationsmittel, umfassend wenigstens eine Verbindung mit mindestens zwei dazu komplementären ionogenen Gruppen, gelöst wird.

Gegenstand der vorliegenden Erfindung ist daher ein wasserlösliches oder wasserdispergierbares polymeres Salz aus:
A) wenigstens einem Polymer PA) mit freien Säuregruppen und einem Neutralisationsmittel, das wenigstens eine Verbindung VA) mit mindestens zwei freien Aminogruppen pro Molekül umfasst, oder
B) wenigstens einem Polymer PB) mit freien Aminogruppen und einem Neutralisationsmittel, das wenigstens eine zwei- oder mehrwertige anorganische Säure und/oder wenigstens eine Verbindung VB) mit mindestens zwei freien Säuregruppen pro Molekül umfasst,
wobei die Verbindungen VA) und VB) zusätzlich wenigstens eine hydrophile Gruppe aufweisen, die ausgewählt ist unter weiteren ionogenen und/oder ionischen Gruppen, zweiwertigen Resten von Polyethern, zweiwertigen Resten von pyrrolidongruppenhaltigen Polymeren und Kombinationen davon.

Im Rahmen der vorliegenden Erfindung umfassen die Ausdrücke Alkyl und Alkylen geradkettige und verzweigte Alkyl- bzw. Alkylengruppen. Vorzugsweise handelt es sich dabei um geradkettige oder verzweigte, C₁- bis C₄₀- und besonders bevorzugt C₂- bis C₃₀-Alkylund -Alkylengruppen.

C₁- bis C₆-Alkyl steht vorzugsweise für Methyl, Ethyl, Isopropyl, n-Butyl, 2-Butyl, sec.-Butyl, tert.-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl etc.

C₂- bis C₆-Alkylen steht für geradkettige und verzweigte C₂- bis C₆-Alkylenreste, bevorzugt C₂- bis C₄-Alkylenreste. Dazu zählen vorzugsweise Ethylen, Propylen, Propan-1,3-diyl, Butan-1,4-diyl, Butan-1,3-diyl, Butan-1,2-diyl, Butan-2,3-diyl, 2-Methylpropan-1,3-diyl, Pentan-1,5-diyl, Pentan-1,4-diyl, Pentan-1,3-diyl, Pentan-1,2-diyl, 1-Methylbutan-1,4-diyl, 2-Methylbutan-1,4-diyl, Hexan-1,6-diyl, Hexan-1,5-diyl, Hexan-1,4-diyl, Hexan-1,3-diyl, Hexan-1,2-diyl etc.

C₆- bis C₄₀-Alkenyl steht vorzugsweise für geradkettige und verzweigte Alkenylgruppen, die einfach, zweifach oder mehrfach ungesättigt sein können. Bevorzugt handelt es sich um C₉- bis C₃₅-, insbesondere um C₁₀- bis C₃₀- und speziell um C12- bis C₂₆-Alkenylgruppen. Dazu zählen insbesondere Hexenyl, Heptenyl, Octenyl, Nonenyl, Decenyl, Undecenyl, Dodecenyl, Tridecenyl, Tetradecenyl, Pentadecenyl, Hexadecenyl, Heptadecenyl, Octadecenyl, Nonadecenyl, Linolyl, Linolenyl, Eläostearyl etc.

Die erfindungsgemäßen polymeren Salze sind z. B. durch eine Neutralisation erhältlich.

Bei der Neutralisation kann ein Polymer PA) mit freien (ungeladenen) Säuregruppen mit einem Neutralisationsmittel umgesetzt werden, das wenigstens eine Verbindung VA) mit mindestens zwei freien Aminogruppen pro Molekül umfasst. Bei der Neutralisation kann auch ein Polymer PB) mit freien (ungeladenen) Aminogruppen mit einem Neutralisationsmittel umgesetzt werden, das wenigstens eine Verbindung VB) mit mindestens zwei freien Säuregruppen pro Molekül umfasst.

Vorzugsweise handelt es sich bei den Säuregruppen der Komponenten PA) und VB) um Carbonsäure und/oder Sulfonsäuregruppen.

Vorzugsweise handelt es sich bei den Aminogruppen der Komponenten PB) und VA) um primäre, sekundäre und/oder tertiäre Aminogruppen, speziell um tertiäre Aminogruppen.

Die erfindungsgemäßen polymeren Salze sind vorzugsweise zumindest teilweise vernetzt. Die Vernetzung erfolgt dabei über Ionenbindung zwischen ionischen Gruppen von mindestens zwei verschiedenen Polymerketten des Polymers PA) oder PB) und wenigstens zwei komplementären ionischen Gruppen einer Komponente VA) oder VB). Gegenstand der Erfindung sind daher auch vernetzte wasserlösliche oder wasserdispergierbare polymere Salze.

Als Polymere PA) und PB) können vorzugsweise Polymere einer Polymerklasse, Gemische ("blends") von Polymeren einer Polymerklasse und Gemische von Polymeren aus zwei oder mehreren Polymerklassen eingesetzt werden. Vorzugsweise sind die Polymere PA) und PB) ausgewählt unter folgenden Klassen:
- Polyurethanen (PA und PB)
- Poly(meth)acrylaten (PA)
- Poly(meth)acrylamiden (PA)
- pyrrolidongruppenhaltigen Polymeren (PA und PB)
und Mischungen davon.

Werden als Polymere PA) und PB) Gemische aus zwei oder mehreren Klassen eingesetzt, so enthalten diese bevorzugt wenigstens ein Polyurethan.

Bevorzugt umfasst die Komponente PA) oder PB) wenigstens ein Polyurethan, welches
a) mindestens ein Polymerisat mit wenigstens zwei aktiven Wasserstoffatomen pro Molekül,
b) mindestens eine Verbindung, die zwei aktive Wasserstoffatome und mindestens eine anionogene und/oder anionische Gruppe pro Molekül aufweist,
c) gegebenenfalls mindestens eine Verbindung mit einem Molekulargewicht im Bereich von 56 bis 500, die zwei aktive Wasserstoffatome pro Molekül enthält, und
d) mindestens ein Diisocyanat
eingebaut enthält.

Bei der Komponente a) handelt es sich bevorzugt um ein Polymerisat mit einem zahlenmittleren Molekulargewicht im Bereich von etwa 300 bis 5 000, bevorzugt etwa 400 bis 4 000, insbesondere 500 bis 3 000. Brauchbare Polymerisate a) sind z. B. Polyesterdiole, Polyetherole, Polysiloxane und Mischungen davon. Polyetherole sind vorzugsweise Polyalkylenglykole, z. B. Polyethylenglykole, Polypropylenglykole, Polytetrahydrofurane etc., Copolymerisate aus Ethylenoxid und Propylenoxid oder Blockcopolymerisate aus Ethylenoxid, Propylenoxid und Butylenoxid, die die Alkylenoxideinheiten statistisch verteilt oder in Form von Blöcken einpolymerisiert enthalten. Geeignet sind auch α,ω-Diaminopolyether, die durch Aminierung von Polyalkylenoxiden mit Ammoniak herstellbar sind. Vorzugsweise werden als Komponente a) Polyesterdiole und Mischungen, die diese enthalten, eingesetzt.

Geeignete Polytetrahydrofurane a) können durch kationische Polymerisation von Tetrahydrofuran in Gegenwart von sauren Katalysatoren, wie z. B. Schwefelsäure oder Fluoroschwefelsäure, hergestellt werden. Derartige Herstellungsverfahren sind dem Fachmann bekannt.

Bevorzugte Polyesterdiole a) weisen ein zahlenmittleres Molekulargewicht im Bereich von etwa 400 bis 5 000, bevorzugt 500 bis 3 000, insbesondere 600 bis 2 000, auf.

Als Polyesterdiole kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure etc., aliphatischer Dicarbonsäuren, wie Adipin säure oder Bernsteinsäure etc., und cycloaliphatischer Dicarbonsäuren, wie 1,2-, 1,3- oder 1,4-Cyclohexandicarbonsäure. Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan.

Bevorzugt sind Polyesterdiole auf Basis von aromatischen und aliphatischen Dicarbonsäuren und aliphatischen Diolen, insbesondere solche, bei denen die aromatische Dicarbonsäure 10 bis 95 Mol-%, insbesondere 40 bis 90 Mol-% des gesamten Dicarbonsäureanteils (Rest aliphatische Dicarbonsäuren) ausmacht.

Besonders bevorzugte Polyesterdiole sind die Umsetzungsprodukte aus Phthalsäure/Diethylenglykol, Isophthalsäure/1,4-Butandiol, Isophthalsäure/Adipinsäure/1,6-Hexandiol, 5-NaSO₃-Isophthalsäure/Phthalsäure/Adipinsäure/1,6-Hexandiol, Adipinsäure/Ethylenglykol, Isophthalsäure/Adipinsäure/Neopentylglykol, Isophthalsäure/Adipinsäure/Neopentylglykol/Diethylenglykol/Dimethylolcyclohexan und 5-NaSO₃-Isophthalsäure/Isophthalsäure/Adipinsäure/Neopentylglykol/Diethylenglykol/Dimethylolcyclohexan, Isophthalsäure/Adipinsäure, Neopentylglykol/Dimethylolcyclohexan.

Bei den Polysiloxanen a) handelt es sich vorzugsweise um eine Verbindung der Formel VII 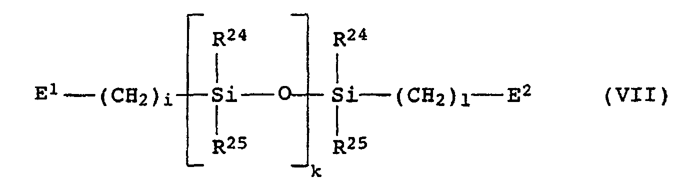 worin
- R²⁴ und R²⁵: unabhängig voneinander für C₁- bis C₄-Alkyl, Benzyl oder Phenyl stehen,
- E¹ und E²: unabhängig voneinander für OH oder NHR²⁶ stehen, wobei R²⁶ für Wasserstoff, C₁- bis C₆-Alkyl oder C₅- bis C₈-Cycloalkyl steht, i und 1 unabhängig voneinander für 2 bis 8 stehen,
- k: für 3 bis 50 steht,
und Mischungen davon.

Geeignete Alkylreste sind z. B. Methyl, Ethyl, n-Propyl, Isopropyl, n-Butyl, t.-Butyl, n-Pentyl, n-Hexyl etc. Geeignete Cycloalkylreste sind z. B. Cyclopentyl, Cyclohexyl, Cycloheptyl, Cyclooctyl etc.

Vorzugsweise stehen R²⁴ und R²⁵ beide für Methyl.

Diese Polysiloxane a) weisen vorzugsweise ein zahlenmittleres Molekulargewicht im Bereich von etwa 300 bis 5 000, bevorzugt 400 bis 3 000, auf.

Bei den Polysiloxanen a) handelt es sich weiterhin vorzugsweise um eine Verbindung der Formel VIII 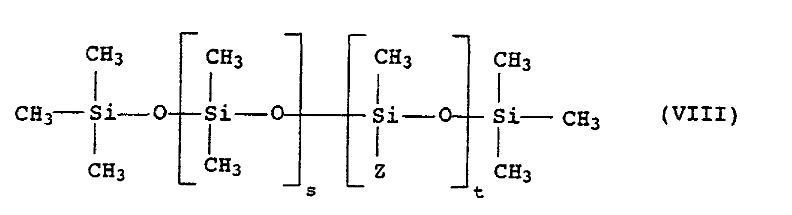 worin
die Reihenfolge der Siloxaneinheiten beliebig ist,
- s: für einen Wert von 5 bis 200, bevorzugt 10 bis 100, steht,
- t: für einen Wert von 1 bis 20, bevorzugt 2 bis 10, steht,
- Z: für einen Rest der Formel -(CH₂)ᵤ-NH₂ steht, worin u für eine ganze Zahl von 1 bis 10, bevorzugt 2 bis 6 steht, oder
Z für einen Rest der Formel -(CH₂)ₓ-NH-(CH₂)_{y}-NH₂ steht, worin x und y unabhängig voneinander für 0 bis 10, bevorzugt 1 bis 6, stehen, wobei die Summe aus x und y für 1 bis 10, bevorzugt 2 bis 6, steht.

Dazu zählen z. B. die MAN- und MAR-Marken der Fa. Hüls sowie die Finish-Marken der Fa. Wacker, z. B. Finish WT 1270.

Geeignete Verbindungen a) sind auch die in der EP-A-227 816 beschriebenen Polydimethylsiloxane, auf die hiermit Bezug genommen wird.

Geeignete Verbindungen b) weisen zwei aktive Wasserstoffatome und mindestens eine ionogene und/oder ionische Gruppe pro Molekül auf, wobei es sich um anionogene, anionische, kationogene oder kationische Gruppen handelt.

Bevorzugte Verbindungen b) mit zwei aktiven Wasserstoffatomen und mindestens einer anionogenen und/oder anionischen Gruppe pro Molekül sind z. B. Verbindungen mit Carboxylat- und/oder Sulfonatgruppen. Als Komponente b) sind 2,2-Hydroxymethyl-alkylcarbonsäuren, wie Dimethylolpropansäure, und Mischungen, die 2,2-Hydroxymethyl-alkylcarbonsäuren, wie Dimethylolpropansäure, enthalten, besonders bevorzugt.

Geeignete Diamine und/oder Diole b) mit anionogenen oder anionischen Gruppen sind Verbindungen der Formel 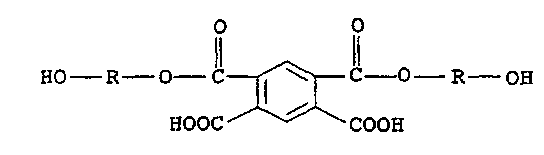 und/oder 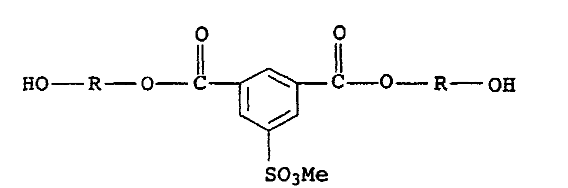 worin R jeweils für eine C₂-C₁₈-Alkylengruppe steht und Me für Na oder K steht.

Als Komponente b) brauchbar sind auch Verbindungen der Formel

H₂N(CH₂)_{w}-NH-(CH₂)ₓ-COO-M⁺

H₂N(CH₂)_{w}-NH-(CH₂)ₓ-SO₃-M⁺

worin w und x unabhängig voneinander für eine ganze Zahl von 1 bis 8, insbesondere 1 bis 6, stehen und M für Li, Na oder K steht, und Verbindungen der Formel

H₂N(CH₂CH₂O)_{y}(CH₂CH(CH₃)O)_{z}(CH₂)_{w}-NH-(CH₂)ₓ-SO₃-M⁺

worin w und x die zuvor angegebenen Bedeutungen besitzen, y und z unabhängig voneinander für eine ganze Zahl von 0 bis 50 stehen, wobei wenigstens eine der beiden Variablen y oder z > 0 ist. Die Reihenfolge der Alkylenoxideinheiten ist dabei beliebig. Die zuletzt genannten Verbindungen weisen vorzugsweise ein zahlenmittleres Molekulargewicht im Bereich von etwa 400 bis 3 000 auf. Eine geeignete Verbindung dieses Typs ist z. B. Poly ESP 520 der Fa. Raschig.

Die Polyurethane können auch Verbindungen b) eingebaut enthalten, die zwei aktive Wasserstoffatome und mindestens eine kationogene und/oder kationische Gruppe, bevorzugt mindestens eine stickstoffhaltige Gruppe, pro Molekül aufweisen. Bevorzugt handelt es sich bei der stickstoffhaltigen Gruppe um eine tertiäre Aminogruppe oder eine quaternäre Ammoniumgruppe. Bevorzugt sind z. B. Verbindungen der allgemeinen Formeln 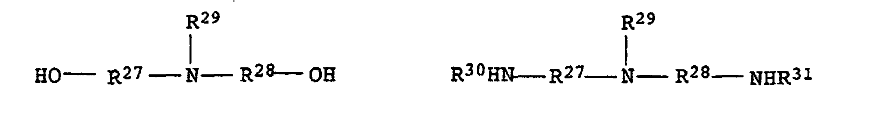 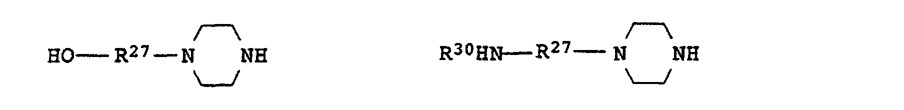 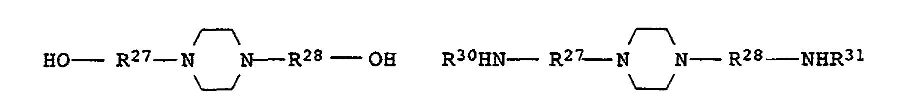 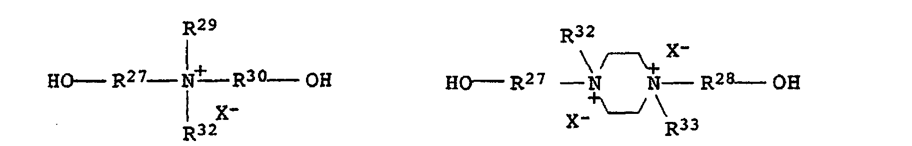 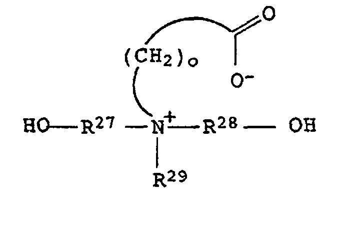 worin
- R²⁷ und R²⁸, die gleich oder verschieden sein können, für -C₂-C₈-Alkylen stehen,
- R²⁹, R³² und R³³, die gleich oder verschieden sein können, für C₁-C₆-Alkyl, Phenyl oder Phenyl-C₁-C₄-alkyl stehen,
   R³⁰ und R³¹, die gleich oder verschieden sein können, für H oder C₁-C₆-Alkyl stehen,
   o für 1, 2 oder 3 steht,
   X^{⊖} für Chlorid, Bromid, Jodid, C₁-C₆-Alkylsulfat oder SO₄²⁻/₂ steht.

Besonders bevorzugt sind N-(C₁- bis C₆-alkyl)diethanolamine, wie Methyldiethanolamin. Diese werden vorzugsweise in Kombination mit Dimethylolpropansäure als Komponente b) eingesetzt.

Als Komponente b) eignen sich auch Gemische, die zwei oder mehrere der zuvor genannten Verbindungen mit anionischen und/oder anionogenen Gruppen, zwei oder mehrere der zuvor genannten Verbindungen mit kationischen und/oder kationogenen Gruppen oder Gemische, die mindestens eine der zuvor genannten Verbindungen mit anionischen oder anionogenen Gruppen und mindestens eine der zuvor genannten Verbindungen mit kationischen oder kationogenen Gruppen enthalten. Bevorzugt werden z. B. Gemische eingesetzt, die Dimethylolpropansäure und N-Methyldiethanolamin enthalten. Nach einer bevorzugten Ausführungsform enthalten die Polyurethane überwiegend oder ausschließlich anionogene und/oder anionische Gruppen als ionogene und/oder ionische Gruppen. Nach einer weiteren bevorzugten Ausführungsform enthalten die Polyurethane überwiegend oder ausschließlich kationogene und/oder kationische Gruppen als ionogene und/oder ionische Gruppen. Bevorzugt enthalten die Polyurethane somit eine Komponente b) eingebaut, die überwiegend, bevorzugt zu mindestens 80 Gew.-%, insbesondere zu mindestens 90 Gew.-%, bezogen auf die Gesamtmenge der Komponente b), entweder anionogene (anionische) Verbindungen oder kationogene (kationische) Verbindungen umfasst.

Bei der Komponente c) handelt es sich bevorzugt um Diole, Diamine, Aminoalkohole, und Mischungen davon. Das Molekulargewicht dieser Verbindungen liegt vorzugsweise in einem Bereich von etwa 56 bis 300. Gewünschtenfalls können bis zu 3 Mol-% der genannten Verbindungen durch Triole oder Triamine ersetzt sein.

Bevorzugt werden als Komponente c) Diole eingesetzt. Brauchbare Diole sind z. B. Ethylenglykol, Propylenglykol, Butylenglykol, Neopentylglykol, Cyclohexandimethylol, Di-, Tri-, Tetra-, Pentaoder Hexaethylenglykol und Mischungen davon. Bevorzugt werden Neopentylglykol und/oder Cyclohexandimethylol eingesetzt.

Geeignete Aminoalkohole c) sind z. B. 2-Aminoethanol, 2-(N-Methylamino)ethanol, 3-Aminopropanol, 4-Aminobutanol, 1-Ethylaminobutan-2-ol, 2-Amino-2-methyl-1-propanol, 4-Methyl-4-aminopentan-2-ol etc.

Geeignete Diamine c) sind z. B. Ethylendiamin, Propylendiamin, 1,4-Diaminobutan, 1,5-Diaminopentan und 1,6-Diaminohexan.

Bei der Komponente d) handelt es sich um übliche aliphatische, cycloaliphatische und/oder aromatische Diisocyanate, wie Tetramethylendiisocyanat, Hexamethylendiisocyanat, Methylendiphenyldiisocyanat, 2,4- und 2,6-Toluylendiisocyanat und deren Isomerengemische, o-, m- und p-Xylylendiisocyanat, 1,5-Naphthylendiisocyanat, 1,4-Cyclohexylendiisocyanat, Dicyclohexylmethandiisocyanat und Mischungen davon, insbesondere Isophorondiisocyanat, Hexamethylendiisocyanat und/oder Dicyclohexylmethandiisocyanat. Gewünschtenfalls können bis zu 3 Mol-% der genannten Verbindungen durch Triisocyanate ersetzt sein.

Die Herstellung der Polyurethane erfolgt durch Umsetzung der Verbindungen der Komponenten a), b) sowie gegebenenfalls c) und/oder zusätzlichen Verbindungen mit kationogenen und/oder kationischen Gruppen mit der Komponente d). Die Temperatur liegt dabei in einem Bereich von etwa 60 bis 140 °C, bevorzugt etwa 70 bis 100 °C. Die Reaktion kann ohne Lösungsmittel oder in einem geeigneten inerten Lösungsmittel oder Lösungsmittelgemisch erfolgen. Geeignete Lösungsmittel sind aprotisch polare Lösungsmittel, z. B. Tetrahydrofuran, Essigsäureethylester, N-Methylpyrrolidon, Dimethylformamid und bevorzugt Ketone, wie Aceton und Methylethylketon. Vorzugsweise erfolgt die Reaktion unter einer Inertgasatmosphäre, wie z. B. unter Stickstoff. Des Weiteren erfolgt die Reaktion vorzugsweise bei Umgebungsdruck oder unter erhöhtem Druck. Die Komponenten werden bevorzugt in solchen Mengen eingesetzt, dass das Verhältnis von NCO-Äquivalent der Verbindungen der Komponente d) zu Äquivalent aktives Wasserstoffatom der Komponenten a), b) und gegebenenfalls c) und/oder weiteren Komponenten in einem Bereich von etwa 0,6:1 bis 1,4:1, bevorzugt 0,8:1 bis 1,2:1, insbesondere 0,9:1 bis 1,1:1, liegt. Gegebenenfalls noch vorhandene freie Isocyanatgruppen der Polyurethane können durch anschließende Umsetzung mit Aminen, vorzugsweise Aminoalkoholen, inaktiviert werden. Geeignete Amine und Aminoalkohole sind die zuvor als Komponente c) genannten, bevorzugt 2-Amino-2-methyl-1-propanol.

Vorzugsweise enthalten die Polyurethane
- 0,5 bis 95 Gew.-%, bevorzugt 1 bis 80 Gew.-%, wenigstens einer Komponente a),
- 1 bis 60 Gew.-%, bevorzugt 3 bis 40 Gew.-%, wenigstens einer Komponente b),
- 0 bis 15 Gew.-%, bevorzugt 0,3 bis 12 Gew.-%, wenigstens einer Komponente c),
- 25 bis 60 Gew.-%, bevorzugt 35 bis 53 Gew.-%, wenigstens einer Komponente d)
einpolymerisiert.

Werden die zuvor beschriebenen Polyurethane erfindungsgemäß als Polymer PA) eingesetzt, so umfasst die Komponente b) wenigstens eine der zuvor beschriebenen Verbindungen mit einer freien Säuregruppe. Der Anteil an Verbindungen mit freien Säuregruppen an der Komponente b) beträgt dann bevorzugt mindestens 51 Gew.-%, besonders bevorzugt mindestens 80 Gew.-%, insbesondere mindestens 90 Gew.-%, bezogen auf die Gesamtmenge der Komponente b). Gewünschtenfalls kann der Rest der Komponente b) zu 100 Gew.-% wenigstens eine der zuvor beschriebenen amingruppenhaltigen Komponenten umfassen.

Werden die zuvor beschriebenen Polyurethane erfindungsgemäß als Polymer PB) eingesetzt, so umfasst die Komponente b) wenigstens eine der zuvor beschriebenen Verbindungen mit mindestens einer primären, sekundären oder tertiären Aminogruppe. Der Anteil an Verbindungen mit freien Aminogruppen an der Komponente b) beträgt dann bevorzugt mindestens 51 Gew.-%, besonders bevorzugt mindestens 80 Gew.-%, insbesondere mindestens 90 Gew.-%, bezogen auf die Gesamtmenge der Komponente b). Gewünschtenfalls kann der Rest der Komponente b) zu 100 Gew.-% wenigstens eine der zuvor beschriebenen säuregruppenhaltigen Komponenten umfassen.

Nach einer weiteren bevorzugten Ausführungsform umfasst die Komponente PA) mindestens ein Polymer, welches
e) wenigstens eine α,β-ethylenisch ungesättigte Mono- und/oder Dicarbonsäure,
f) wenigstens ein α,β-ethylenisch ungesättigtes Monomer, das ausgewählt ist unter Estern α,β-ethylenisch ungesättigter Monound/oder Dicarbonsäuren mit geradkettigen und/oder verzweigten C₁- bis C₆-Alkanolen, Amiden α,β-ethylenisch ungesättigter Mono- und/oder Dicarbonsäuren mit Mono- und Dialkylaminen mit geradkettigen und/oder verzweigten C₁- bis C₆-Alkylresten und Mischungen davon,
g) gegebenenfalls wenigstens ein weiteres, von e) und f) verschiedenes Monomer mit mindestens einer α,β-ethylenisch ungesättigten Doppelbindung,
einpolymerisiert enthält.

Geeignete Monomere e) sind α,β-ethylenisch ungesättigte Mono- und/oder Dicarbonsäuren und deren Halbester und Anhydride, wie Acrylsäure, Methacrylsäure, Fumarsäure, Maleinsäure, Itaconsäure, Crotonsäure, Maleinsäureanhydrid, Monobutylmaleat etc., und Gemische davon. Bevorzugt werden Acrylsäure, Methacrylsäure und Gemische davon eingesetzt.

Geeignete Monomere f) sind die Ester α,β-ethylenisch ungesättigter Mono- und Dicarbonsäuren mit geradkettigen und/oder verzweigter C₁-C₆-Alkanolen, bevorzugt C₂-C₄-Alkanolen, z. B. die Ester der Acrylsäure und/oder Methacrylsäure mit Methanol, Ethanol, n-Propanol, Isopropanol, n-Butanol, sec.-Butanol, tert.-Butanol, n-Pentanol, 2-Methylbutanol, n-Hexanol etc.

Geeignete Monomere f) sind weiterhin Amide α,β-ethylenisch ungesättigter Mono- und Dicarbonsäuren mit Mono- und Dialkylaminen mit geradkettigen und/oder verzweigten Alkylresten, die 1 bis 6 Kohlenstoffatome, bevorzugt 2 bis 4 Kohlenstoffatome, pro Alkylrest aufweisen. Dazu zählen z. B. N-C₁- bis C₆-Alkyl(meth)acrylamide, wie N-Methyl(meth)acrylamid, N-Ethyl(meth)- acrylamid, N-(n-Propyl)(meth)acrylamid, N-Isopropyl(meth)acrylamid, N-(n-Butyl)(meth)acrylamid, N-(tert.-Butyl)(meth)acrylamid, N-(n-Pentyl)(meth)acrylamid, N-(n-Hexyl)(meth)acrylamid, N,N-Dimethyl(meth)acrylamid, N,N-Diethyl(meth)acrylamid etc.

Vorzugsweise umfasst die Komponente f) wenigstens einen Ester einer α,β-ethylenisch ungesättigten Mono- und/oder Dicarbonsäure mit einem linearen C₂- bis C₆-Alkanol. Insbesondere handelt es sich um einen Ester der Acrylsäure und/oder Methacrylsäure mit Ethanol, n-Propanol, n-Butanol, n-Pentanol und n-Hexanol. Speziell umfasst die Komponente f) n-Butylacrylat und/oder n-Butylmethacrylat.

Vorzugsweise enthalten die Polymere als Komponente f) mindestens ein lineares C₁- bis C₆-Alkyl(meth)acrylat und/oder -acrylamid, insbesondere n-Butyl(meth)acrylat und/oder n-Butyl(meth)acrylamid, und mindestens ein verzweigtes C₁- bis C₆-Alkyl(meth)acrylat und/oder -acrylamid, insbesondere tert.-Butyl(meth)acrylat und/oder tert.-Butyl(meth)acrylamid, einpolymerisiert.

Geeignete Monomere g) sind N-Vinylamide, wie N-Vinylformamid, N-Vinylacetamid, N-Vinylpropionamid etc. Bevorzugt wird N-Vinylformamid eingesetzt.

Geeignete Monomere g) sind weiterhin N-Vinyllactame und deren Derivate, die z. B. einen oder mehrere C₁-C₆-Alkylsubstituenten, wie Methyl, Ethyl, n-Propyl, Isopropyl, n-Butyl, sec.-Butyl, tert.-Butyl etc. aufweisen können. Dazu zählen z. B. N-Vinylpyrrolidon, N-Vinylpiperidon, N-Vinylcaprolactam, N-Vinyl-5-methyl-2-pyrrolidon, N-Vinyl-5-ethyl-2-pyrrolidon, N-Vinyl-6-methyl-2-piperidon, N-Vinyl-6-ethyl-2-piperidon, N-Vinyl-7-methyl-2-caprolactam, N-Vinyl-7-ethyl-2-caprolactam etc.

Geeignete Monomere g) sind weiterhin primäre Amide der zuvor genannten α,β-ethylenisch ungesättigten Monocarbonsäuren, wie Acrylamid, Methacrylamid, Ethacrylamid etc.

Geeignete Monomere g) sind weiterhin vinyl- und allylsubstituierte heteroaromatische Verbindungen, wie 2- und 4-Vinylpyridin, -Allylpyridin, und bevorzugt N-Vinylheteroaromaten, wie N-Vinylimidazol, N-Vinyl-2-methylimidazol etc.

Geeignete Monomere g) sind weiterhin Vinylformiat, Vinylacetat, Vinylpropionat, Vinyl-n-butyrat, Vinylstearat, Vinyllaurat, Styrol, α-Methylstyrol, o-Chlorstyrol, Vinyltoluole, Vinylchlorid, Vinylidenchlorid, Ethylen, Propylen, Butadien, Isopren, Chloropren, Methyl-, Ethyl-, Butyl-, Dodecylvinylether etc.

Bevorzugte Monomere g) sind die Ester α,β-ethylenisch ungesättigter Mono- und Dicarbonsäuren mit geradkettigen und/oder verzweigten C₇- bis C₂₀-Alkanolen, bevorzugt C₇- bis C₁₂-Alkanolen, z. B. die Ester der Acrylsäure und/oder Methacrylsäure mit n-Heptanol, n-Octanol, 2-Ethylhexanol, n-Nonanol, n-Decanol, n-Dodecanol etc.

Bevorzugte Monomere g) sind weiterhin die Amide α,β-ethylenisch ungesättigter Mono- und Dicarbonsäuren mit Mono- und Dialkylaminen mit geradkettigen und/oder verzweigten Alkylresten, die 7 bis 20 Kohlenstoffatome, bevorzugt 7 bis 12 Kohlenstoffatome, aufweisen. Dazu zählen z. B. N-Isopropyl(meth)acrylamid, N-(tert.-Butyl)(meth)acrylamid, N-Ethylhexyl(meth)acrylamid, N,N-Diisopropyl(meth)acrylamid etc.

Bevorzugt enthält das Polymerisat PA)
- 1 bis 60 Gew.-%, bevorzugt 5 bis 50 Gew.-%, insbesondere 10 bis 40 Gew.-%, wenigstens einer Komponente e),
- 10 bis 99 Gew.-%, bevorzugt 20 bis 95 Gew.-%, insbesondere 30 bis 90 Gew.-%, wenigstens einer Komponente f),
- 0 bis 89 Gew.-%, bevorzugt 0,1 bis 50 Gew.-%, insbesondere 1 bis 40 Gew.-%, wenigstens einer Komponente g),
einpolymerisiert.

Nach einer bevorzugten Ausführungsform enthält das Polymerisat PA)
- 10 bis 40 Gew.-%, bevorzugt 15 bis 35 Gew.-%, wenigstens einer Komponente e),
- 30 bis 90 Gew.-%, bevorzugt 35 bis 80 Gew.-%, wenigstens einer Komponente f), umfassend mindestens ein geradkettiges C₁bis C₆-Alkyl(meth)acrylat und/oder C₁- bis C₆-Alkyl(meth)-acrylamid mit geradkettigen Alkylresten und mindestens ein verzweigtes C₁- bis C₆-Alkyl(meth)acrylat und/oder C₁- bis C₆-Alkyl(meth)acrylamid mit verzweigten Alkylresten,
- 0 bis 60 Gew.-%, bevorzugt 0,1 bis 40 Gew.-%, wenigstens eine verzweigten C₄- bis C₂₀-Alkyl(meth)acrylats und/oder C₄bis C₂₀-Mono- oder Dialkyl(meth)acrylamids
einpolymerisiert.

Die Herstellung dieser Polymere PA) erfolgt nach üblichen, dem Fachmann bekannten Verfahren. Dazu zählt die Polymerisation in Masse und vorzugsweise die Emulsions-, Suspensions- und Lösungspolymerisation. Die Polymerisationstemperatur beträgt in der Regel 30 bis 120 °C, bevorzugt 40 bis 100 °C. Das Polymerisationsmedium kann für die Lösungspolymerisation sowohl nur aus einem organischen Lösungsmittel als auch aus Mischungen aus Wasser und mindestens einem wassermischbaren, organischen Lösungsmittel bestehen. Bevorzugte organische Lösungsmittel sind z. B. Alkohole, wie Methanol, Ethanol, n-Propanol, Isopropanol, n-Butanol, Ketone, wie Aceton und Methylethylketon, Tetrahydrofuran etc. Bei der Emulsions- und Suspensionspolymerisation wird vorzugsweise Wasser als Lösungsmittel eingesetzt. Die Polymerisation kann sowohl als Batchprozess als auch in Form eines Zulaufverfahrens, einschließlich Monomerenzulauf, Stufen- und Gradientenfahrweise, durchgeführt werden. Bevorzugt ist im Allgemeinen das Zulaufverfahren, bei dem man gegebenenfalls einen Teil des Polymerisationsansatzes vorlegt, auf die Polymerisationstemperatur erhitzt und anschließend den Rest des Polymerisationsansatzes, üblicherweise über einen oder auch mehrere, räumliche getrennte Zuläufe, kontinuierlich, stufenweise oder unter Überlagerung eines Konzentrationsgefälles unter Aufrechterhaltung der Polymerisation der Polymerisationszone zuführt.

Als Initiatoren für die radikalische Polymerisation kommen Azoverbindungen in Frage, die für die radikalische Polymerisation geeignet sind. Dazu zählen aliphatische oder cycloaliphatische Azoverbindungen, z. B. 2,2'-Azobis (isobutyronitril), 2,2'-Azobis(2-methylbutyronitril), 2,2'-Azobis(2,4-dimethylvaleronitril), 1,1'-Azobis(1-cyclohexancarbonitril), 2-(Carbamoylazo)isobutyronitril, 4,4'-Azobis(4-cyanovaleriansäure) und deren Alkalimetallund Ammoniumsalze, z. B. das Natriumsalz, Dimethyl-2,2'-azobisisobutyrat, 2,2'-Azobis[2-(2-imidazolin-2-yl)propan], 2,2'-Azobis(2-amidinopropan) und die Säureadditionssalze der beiden zuletzt genannten Verbindungen, z. B. die Dihydrochloride.

Ferner kommen als Initiatoren Wasserstoffperoxid, Hydroperoxide in Kombination mit Reduktionsmitteln und Persalze in Frage. Geeignete Hydroperoxide sind beispielsweise t-Butylhydroperoxid, t-Amylhydroperoxid, Cumolhydroperoxid und Pinanhydroperoxid jeweils in Kombination mit beispielsweise einem Salz der Hydroxymethansulfinsäure, einem Eisen (II)-Salz oder Ascorbinsäure. Geeignete Persalze sind insbesondere Alkalimetallperoxidisulfate.

Die verwendete Initiatormenge, bezogen auf die Monomere, liegt im Allgemeinen in einem Bereich von etwa 0,02 bis 15 Mol-%, vorzugsweise 0,05 bis 3 Mol-%.

Sind niedrigere Molekulargewichte erwünscht, so können diese durch Zusatz eines Reglers zum Polymerisationsansatz eingestellt werden. Als Regler eignen sich beispielsweise Aldehyde, wie Formaldehyd, Acetaldehyd, Propionaldehyd, n-Butyraldehyd und Isobutyraldehyd, Ameisensäure, Ammoniumformiat, Hydroxylammoniumsulfat und Hydroxylammoniumphosphat. Weiterhin können Regler eingesetzt werden, die Schwefel in organisch gebundener Form enthalten, wie Di-n-butylsulfid, Di-n-octylsulfid, Diphenylsulfid etc., oder Regler, die Schwefel in Form von SH-Gruppen enthalten, wie n-Butylmercaptan, n-Hexylmercaptan oder n-Dodecylmercaptan. Geeignet sind auch wasserlösliche, schwefelhaltige Polymerisationsregler, wie beispielsweise Hydrogensulfite und Disulfite. Weiterhin eignen sich als Regler Allylverbindungen, wie Allylalkohol oder Allylbromid, Benzylverbindungen, wie Benzylchlorid oder Alkylhalogenide, wie Chloroform oder Tetrachlormethan.

Geeignete Emulgatoren für die Emulsionspolymerisation sind die dafür üblichen und dem Fachmann bekannten anionischen, kationischen und nichtionischen Tenside. Gewünschtenfalls kann die Emulsionspolymerisation in Gegenwart üblicher Schutzkolloide durchgeführt werden. Geeignete Emulgatoren und Schutzkolloide sind z. B. in Houben-Weyl, Methoden der Organischen Chemie, Bd. XIV/1, Makromolekulare Stoffe, Georg-Thieme-Verlag, Stuttgart 1961, S. 192-208 und 411-420 beschrieben.

Gewünschtenfalls setzt man dem Reaktionsgemisch im Anschluss an die Polymerisationsreaktion einen oder mehrere Polymerisationsinitiatoren zu und erhitzt die Polymerlösung, z. B. auf die Polymerisationstemperatur oder auf Temperaturen oberhalb der Polymerisationstemperatur, um die Polymerisation zu vervollständigen. Geeignet sind die oben angegebenen Azoinitiatoren, aber auch alle anderen üblichen, für eine radikalische Polymerisation in wässriger Lösung geeignete Initiatoren, beispielsweise Peroxide, Hydroperoxide, Peroxodisulfate, Percarbonate, Peroxoester und Wasserstoffperoxid. Hierdurch wird die Polymerisationsreaktion zu einem höheren Umsatz, wie z. B. von 99,9 %, geführt. Die bei der Polymerisation entstehenden Lösungen können gegebenenfalls durch ein dem Stand der Technik entsprechendes Trocknungsverfahren in feste Pulver überführt werden. Bevorzugte Verfahren sind beispielsweise die Sprühtrocknung, die Sprühwirbelschichttrocknung, die Walzentrocknung und die Bandtrocknung. Ebenfalls anwendbar sind die Gefriertrocknung und die Gefrierkonzentrierung. Gewünschtenfalls kann das Lösungsmittel auch durch übliche Methoden, z. B. Destillation bei verringertem Druck, teilweise oder vollständig entfernt und gegebenenfalls gegen das für die folgende Umsetzung eingesetzte Lösungsmittel ausgetauscht werden.

Bevorzugte Polymere PA) oder PB) sind weiterhin pyrrolidongruppenhaltige Polymere, die z. B. durch Umsetzung eines Monomerengemisches erhältlich sind, welches
h) Itaconsäure und/oder ein Derivat davon, und
i) wenigstens ein Diamin der allgemeinen Formel I

   H₂N-A-NHR¹ (I)

   worin
   - R¹: für Wasserstoff oder C₁- bis C₄-Alkyl steht,
   - A: für einen C₂- bis C₂₀-Alkylenrest steht, der durch wenigstens eine oder mehrere nicht benachbarte, gleiche oder verschiedene Gruppen -NR²- unterbrochen sein kann, wobei R² für Wasserstoff, C₁- bis C₄-Alkyl, C₅- bis C₈-Cycloalkyl, Phenyl oder Phenyl-C₁-C₄-alkyl steht,
enthält.

Nach einer bevorzugten Ausführungsform umfasst die Komponente PA) oder PB) der erfindungsgemäßen polymeren Salze mindestens ein pyrrolidongruppenhaltiges Polymer.

Bevorzugte Verbindungen h) sind z. B. Itaconsäure, Itaconsäureanhydrid, Itaconsäure-(C₁-C₆-)dialkylester, wie Itaconsäuredimethylester, Itaconsäurediethylester, Itaconsäuredihalogenide, wie Itaconsäuredichlorid, etc.

Bevorzugte Verbindungen i) sind z. B. Verbindungen der Formel I, worin A für 1,2-Ethylen, 1,3-Propylen, 1,4-Butylen, 2,3-Butylen, Pentamethylen, Hexamethylen, Heptamethylen, Octamethylen, Nonamethylen, Decamethylen, -CH₂-O-CH₂-, 1,4-Cyclohexylen, 1,3-Cyclohexylen etc. steht.

Vorzugsweise steht in der Formel I der Rest R¹ für Wasserstoff, Methyl, Ethyl, n-Propyl, Isopropyl, n-Butyl oder tert.-Butyl.

Vorzugsweise ist die Verbindung i) ausgewählt unter Ethylendiamin, 4,4'-Biscyclohexylmethandiamin, 1,4-Butylendiamin, 2,3-Butylendiamin, Hexamethylendiamin, 1,4-Cyclohexylendiamin, 1,3-Cyclohexylendiamin und Gemischen davon.

Vorzugsweise enthalten die pyrrolidongruppenhaltigen Polymere wenigstens ein weiteres Monomer k) eingebaut, das ausgewählt ist unter Aminen, die zwei oder mehrere primäre und/oder sekundäre Aminogruppen und zusätzlich wenigstens eine tertiäre Aminogruppe aufweisen. Dazu zählen z. B. die zuvor als Komponente b) genannten Amine der Formel 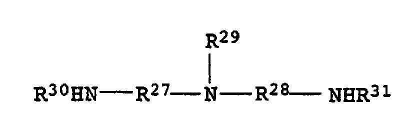 worin R²⁷, R²⁸, R²⁹, R³⁰ und R³¹ die zuvor angegebenen Bedeutungen besitzen.

Bevorzugte Monomere k) sind z. B. N-Methyldipropylentriamin, N-Ethyldiethylentriamin, N-Methyldiethylentriamin, N-Ethyldipropylentriamin und Mischungen davon.

Die Umsetzung von Itaconsäure oder ihren Derivaten mit Aminogruppen enthaltenden monomeren Verbindungen zu Pyrrolidonstrukturen ist prinzipiell bekannt. Man arbeitet vorteilhafterweise hierbei unter einer Inertgasatmosphäre, z. B. unter Stickstoff, in einem Lösungsmittel, vorzugsweise Wasser, und bei Temperaturen von etwa 90 bis 120 °C.

Die sich anschließende Polykondensationsreaktion zur Erzeugung von Polyestern und/oder Polyamiden verläuft in der Regel bei Temperaturen von 100 bis 300 °C, insbesondere 150 bis 250 °C. Aus der ersten Stufe herrührendes Lösungsmittel, z. B. Wasser, wird zweckmäßigerweise vorher entfernt, beispielsweise durch Destillation. Bei der Polykondensation gebildetes Wasser, welches meist als Dampf anfällt, wird ebenfalls zweckmäßigerweise entfernt, entweder während der Polykondensation fortlaufend oder im Anschluss daran. Man arbeitet bei Normaldruck, zweckmäßigerweise unter einer Inertgasatmosphäre, oder bei erhöhtem Druck, etwa bis 25 bar. Die Umsetzung ist in der Regel innerhalb von 2 bis 10 Stunden beendet.

Die Polykondensationsreaktion kann durch Verwendung von Katalysatoren in den hierbei üblichen Mengen beschleunigt werden. Hierzu eignen sich vor allem Mineralsäuren oder saure Salze von Mineralsäuren, z. B. Orthophosphorsäure, Alkalimetalldihydrogenphosphate oder Alkalimetallhydrogensulfate. Auch Schwermetallsalze von Fettsäure, wie Zinnoctanoat, können hierzu eingesetzt werden. Die im Produkt verbleibenden sauren Katalysatoren können mit üblichen Basen neutralisiert werden.

Die zuvor genannten pyrrolidongruppenhaltigen Polymere können vorzugsweise als säuregruppenhaltiges Polymer PA) der erfindungsgemäßen polymeren Salze eingesetzt werden. Zur Herstellung von pyrrolidongruppenhaltigen Polymeren PA) mit freien Säuregruppen können die Komponenten h), i) und gegebenenfalls k) in solchen Mengenverhältnissen eingesetzt werden, dass die - gegebenenfalls derivatisierten - Säuregruppen der Komponente h) in einem molaren Überschuss gegenüber den Aminogruppen der Komponenten i) und, falls vorhanden, k) vorliegen. Bevorzugt beträgt der molare Überschuss etwa 1 bis 25 Mol-%.

Derivatisierte Säuregruppen können abschließend durch Verseifen bzw. Hydrolyse nach üblichen Verfahren in die freien Säuregruppen überführt werden.

Die zuvor genannten pyrrolidonhaltigen Polymere können auch vorzugsweise als aminogruppenhaltiges Polymer PB) der erfindungsgemäßen polymeren Salze eingesetzt werden. Zur Herstellung von pyrrolidongruppenhaltigen Polymeren PB) mit freien Aminogruppen können die Komponenten h), i) und gegebenenfalls k) in solchen Mengenverhältnissen eingesetzt werden, dass die Aminogruppen der Komponenten i) und, falls vorhanden, k) in einem molaren Überschuss gegenüber den - gegebenenfalls derivatisierten - Säuregruppen der Komponente h) vorliegen. Bevorzugt beträgt der molare Überschuss etwa 1 bis 25 Mol-%.

Geeignete pyrrolidongruppenhaltige Polymere und Verfahren zu ihrer Herstellung sind z. B. in der DE-A-43 33 238 beschrieben, auf die hier in vollem Umfang Bezug genommen wird.

Nach einer bevorzugten Ausführungsform erfolgt die Herstellung der erfindungsgemäßen polymeren Salze durch Umsetzung wenigstens eines der zuvor genannten Polymere PA) mit einem Neutralisationsmittel, das wenigstens eine Verbindung VA) mit mindestens zwei freien Aminogruppen pro Molekül aufweist.

Bevorzugt umfasst die Komponente VA) wenigstens ein Polyamin der Formel II 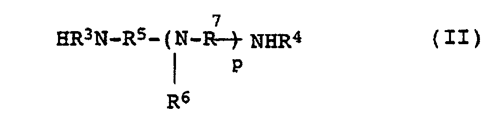 worin
- p: für eine ganze Zahl von 0 bis 4 steht,
- R³ und R⁴: unabhängig voneinander für Wasserstoff, C₁- bis C₄₀-Alkyl oder C₆- bis C₄₀-Alkenyl stehen, wobei die Alkyl- und Alkenylreste wenigstens eine ionogene und/oder ionische Gruppe tragen können, die ausgewählt ist unter -COOY, -SO₃Y und -PO₃Y, wobei Y für H, Li, Na, K oder Ammonium steht, wobei für p = 0 wenigstens einer der Reste R³ oder R⁴ für einen C₁bis C₄₀-Alkyl- oder C₆- bis C₄₀-Alkenylrest steht, der wenigstens eine ionogene und/oder ionische Gruppe trägt,
- R⁵ und R⁷: für einen C₂- bis C₆-Alkylenrest stehen, wobei für p > 1 die Reste R⁷ unabhängig unter C₂- bis C₆-Alkylenresten ausgewählt sind,
- R⁶: für C₁- bis C₆-Alkyl, C₅- bis C₈-Cycloalkyl, Phenyl oder Phenyl-C₁-C₄-alkyl steht, wobei für p > 1 die Reste R₆ unabhängig unter diesen Bedeutungen ausgewählt sind.

Weist das Polyamin der Formel II mehrere Wiederholungseinheiten (̵N(R⁶)-R⁷)̵ₚ auf, so können diese gleiche oder verschiedene Bedeutungen haben.

Bevorzugt steht p für 1, 2 oder 3, insbesondere für 1 oder 2.

Wenn p für 0 steht, so stehen bevorzugt die Reste R³ und R⁴ unabhängig für einen C₁- bis C₄₀-Alkyl- oder C₆- bis C₄₀-Alkenylrest, der jeweils wenigstens eine ionogene und/oder ionische Gruppe trägt.

Bevorzugt stehen R³ und R⁴ unabhängig voneinander für Wasserstoff, C₁- bis C₃₀-Alkyl, bevorzugt C₁- bis C₁₂-Alkyl, insbesondere C₁bis C₈-Alkyl oder einen Rest der Formel 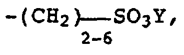 wobei Y für H, Li, Na, K oder Ammonium steht.

Insbesondere stehen R³ und R⁴ unabhängig voneinander für Wasserstoff, Methyl, Ethyl, n-Propyl, Isopropyl, n-Butyl, sec.-Butyl, tert.-Butyl, n-Pentyl, n-Hexyl. Speziell stehen R³ und R⁴ beide für Wasserstoff.

Bevorzugt steht R⁵ für einen C₂- bis C₄-Alkylenrest.

Bevorzugt steht R⁶ für Methyl, Ethyl, n-Propyl, Isopropyl, n-Butyl, sec.-Butyl, tert.-Butyl, Cyclohexyl, Phenyl oder Benzyl.

Bevorzugt steht R⁷ für einen C₂- bis C₄-Alkylenrest.

Das Polyamin der Formel II steht vorzugsweise für Diethylentriamin, N-Methyldiethylentriamin, N-Ethyldiethylentriamin, N,N,N',N'-Tetramethyldiethylentriamin, N,N,N',N'-Tetraethyldiethylentriamin, Dipropylentriamin, N-Methyldipropylentriamin, N-Ethyldipropylentriamin, N,N'-Bis(3-aminopropyl)-butan-1,4-diamin, Triethylentetramin, Tetraethylenpentamin und Mischungen davon. Besonders bevorzugt handelt es sich bei dem Polyamin der Formel II um N-Methyldipropylentriamin.

weiterhin bevorzugt umfasst die Komponente VA) wenigstens einen Diaminopolyether der Formel III

HR⁸N-R¹⁰-O-(CH₂CH₂O)_{q}(C₃H₆O)ᵣ-R¹¹-NHR⁹ (III)

worin
- R⁸ und R⁹: unabhängig voneinander für Wasserstoff, C₁- bis C₄₀-Alkyl oder C₆- bis C₄₀-Alkenyl stehen, wobei die Alkyl- und Alkenylreste gegebenenfalls wenigstens eine ionogene und/oder ionische Gruppe tragen können, die ausgewählt ist unter -COOY, -SO₃Y und -PO₃Y, wobei Y für H, Li, Na, K oder Ammonium steht,
- R¹⁰ und R¹¹: unabhängig voneinander für einen C₂- bis C₆-Alkylenrest stehen,
die Reihenfolge der Alkylenoxideinheiten beliebig ist und q und r unabhängig voneinander für eine ganze Zahl von 0 bis 100 stehen, wobei die Summe aus q und r in einem Bereich von 5 bis 100 liegt.

Bevorzugt stehen R⁸ und R⁹ unabhängig voneinander für Wasserstoff, C₁- bis C₃₀-Alkyl, bevorzugt C₁- bis C₁₂-Alkyl, insbesondere C₁bis C₈-Alkyl oder einen Rest der Formel -(CH₂)₂₋₆-SO₃Y, wobei Y für H, Li, Na, K oder Ammonium steht.

Insbesondere stehen R⁸ und R⁹ unabhängig voneinander für Wasserstoff, Methyl, Ethyl, n-Propyl, Isopropyl, n-Butyl, sec.-Butyl, tert.-Butyl, n-Pentyl, n-Hexyl. Speziell stehen R² und R⁴ beide für Wasserstoff.

Bevorzugt stehen R¹⁰ und R¹¹ unabhängig voneinander für einen C₂bis C₄-Alkylenrest, insbesondere einen C₂- bis C₃-Alkylenrest.

Geeignete Verbindungen der Formel III sind α,ω-Diaminopolyether, die durch Aminierung von Polyalkylenoxiden mit Ammoniak herstellbar sind. Derartige Polyether und Herstellungsverfahren sind dem Fachmann bekannt. Bevorzugt handelt es sich bei den Polyethern um die zuvor als Komponente a) genannten Polyetherole mit einem zahlenmittleren Molekulargewicht im Bereich von etwa 300 bis 5 000, bevorzugt etwa 400 bis 4 000.

Weiterhin bevorzugt umfasst die Komponente VA) wenigstens ein alkoxyliertes Amin der Formel IV

R¹²R¹⁴N-R¹⁶-NR¹³R¹⁵ (IV)

worin
- R¹² und R¹³: unabhängig voneinander für C₁- bis C₄₀-Alkyl, C₆- bis C₄₀-Alkenyl, Hydroxyalkyl oder einen Rest der Formel -(CH₂CH₂O)ₘ(C₃H₆O)ₙ-H stehen, wobei die Reihenfolge der Alkylenoxideinheiten beliebig ist und m und n unabhängig voneinander für eine ganze Zahl von 0 bis 30 stehen, wobei die Summe aus m und n in einem Bereich von 1 bis 30 liegt,
- R¹⁴ und R¹⁵: unabhängig voneinander ausgewählt sind unter den für R¹² und R¹³ angegebenen Bedeutungen und C₂- bis C₆-Alkylresten mit einer ionogenen oder ionischen Gruppe, die ausgewählt ist unter -COOY, -SO₃Y und -PO₃Y, wobei Y für H, Li, Na, K oder Ammonium steht, und
- R¹⁶: für einen C₂- bis C₆-Alkylenrest steht.

Wenn in der Formel IV die Reste R¹², R¹⁴ sowie gegebenenfalls R¹³ und/oder R¹⁵ für Alkoxylatreste stehen, so können diese jeweils gleiche oder verschiedene Bedeutungen haben.

Bevorzugt stehen m und n für jeden der Alkoxilatreste unabhängig voneinander für eine ganze Zahl von 0 bis 20, bevorzugt 0 bis 10, wobei die Summe aus m und n jeweils in einem Bereich von 1 bis 20, bevorzugt 2 bis 10, liegt.

Vorzugsweise weist das alkoxylierte Amin der Formel IV insgesamt 1 bis 50, besonders bevorzugt 2 bis 40, insbesondere 3 bis 30, speziell 3 bis 20, eingebaute Alkylenoxideinheiten auf.

Bevorzugt sind die Reste R¹² und R¹³ ausgewählt unter C₁- bis C₂₂-Alkyl und C₆- bis C₂₂-Alkenyl, insbesondere C₈- bis C₂₂-Alkyl und C8- bis C₂₂-Alkenyl.

Bevorzugt steht wenigstens einer der Reste R¹⁴ und/oder R¹⁵ für einen geradkettigen oder verzweigten C₈- bis C₄₀-Alkyl- oder C₈bis C₄₀-Alkenylrest. Vorzugsweise handelt es sich dabei um geradkettige und verzweigte C₉- bis C₃₅-, besonders bevorzugt C₁₀- bis C₃₀- und speziell C₁₂- bis C₂₆-Alkyl- und -Alkenylreste.

R¹² und/oder R¹⁴ stehen bevorzugt für 1-Octyl, 1-Nonyl, 1-Decyl, 1-Undecyl, 1-Undec-10-enyl, 1-Tridecyl, 1-Tetradecyl, 1-Pentadecyl, 1-Hexadecyl, 1-Heptadecyl, 1-Octadecyl, 1-Octadeca-9,12-dienyl, 1-Nonadecyl, 1-Eicosyl, 1-Eicos-9-enyl, 1-Heneicosyl, 1-Docosyl und insbesondere für Oleyl und 1-Hexadecyl (Cetyl) oder von aus natürlich vorkommenden Fettsäuren durch formales Entfernen der Carbonsäurefunktion abgeleitete Alkylreste, wobei es sich bei den Fettsäuren z. B. um Talgfettsäuren, die überwiegend gesättigte und ungesättigte C₁₄-, C₁₆- und C₁₈-Alkylreste enthalten oder Kokosfette, die gesättigte, einfach und zweifach ungesättigte C8-bis C₂₂-, vorzugsweise C₁₂- bis C₁₄-Alkylfette enthalten, handelt.

Insbesondere steht einer der Reste R¹⁴ und/oder R¹⁵ für einen der zuvor genannten C₈- bis C₄₀-Alkyl- oder C₈- bis C₄₀-Alkenylreste und der andere für einen der zuvor genannten Alkylenoxidreste.

Bevorzugt steht in der Formel IV der Rest R¹⁶ für einen C₂- bis C₄-Alkylenrest.

Insbesondere handelt es sich um ein alkoxyliertes Amin der Formel IV, worin
- R¹², R¹³ und R¹⁵: unabhängig voneinander für Hydroxyethyl oder einen Rest der Formel -(CH₂CH₂O)ₘ-H stehen, wobei m jeweils für eine ganze Zahl von 1 bis 10 steht und die Gesamtzahl der Ethylenoxideinheiten der Reste R¹², R¹³ und R¹⁵ in einem Bereich von 3 bis 20, bevorzugt 4 bis 15, liegt, und
- R¹⁴: für einen C₈- bis C₂₀-Alkyl- oder Alkenylrest steht.

Vorzugsweise steht der Rest R¹⁴ für einen Oleyl- oder Talgrest.

Geeignete alkoxylierte Amine der Formel IV sind z. B. die Dinoramox®-Marken der Firma Ceca und insbesondere Dinoramox® S3 oder S7, d. h. ethoxylierte n-Talg-Propylendiamine mit 3 bzw. 7 Ethylenoxideinheiten. Geeignet sind weiterhin hydroxyethylierte oder ethoxylierte Oleyl-Propylendiamine mit 3 bis 7, insbesondere mit 5 Ethylenoxideinheiten.

Weiterhin bevorzugt handelt es sich bei der Verbindung VA) um ein Diaminopolyethersiloxan der Formel V, das ausgewählt ist unter
- Polysiloxanen mit Wiederholungseinheiten der allgemeinen Formel V.I 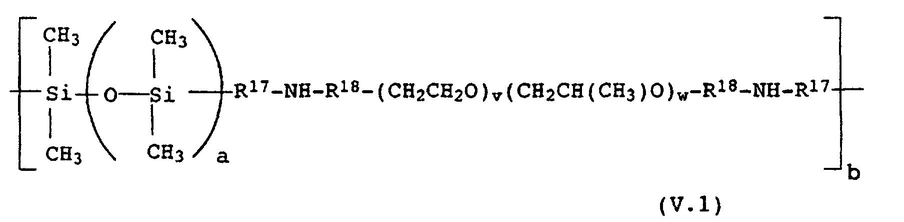 worin
   - a: für eine ganze Zahl von 0 bis 100 steht,
   - b: für eine ganze Zahl von 1 bis 8 steht,
   - R¹⁷ und R¹⁸: unabhängig voneinander für C₁- bis C₈-Alkylen stehen,
   die Reihenfolge der Alkylenoxideinheiten beliebig ist und v und w unabhängig voneinander für eine ganze Zahl von 0 bis 200 stehen, wobei die Summe aus v und w > 0 ist,
- Polysiloxanen der allgemeinen Formel V.2 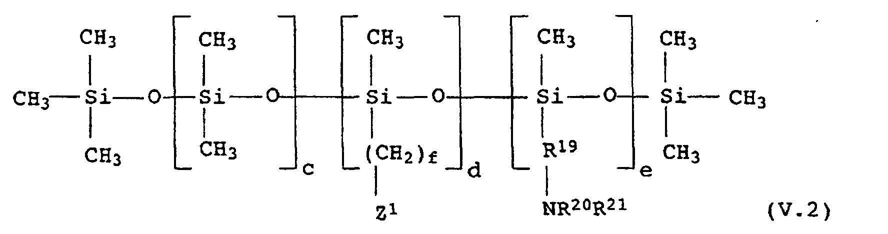 worin
   - R¹⁹: für einen C₁- bis C₈-Alkylenrest steht,
   - R²⁰ und R²¹: unabhängig voneinander für Wasserstoff, C₁- bis C₈-Alkyl oder C₅- bis C₈-Cycloalkyl stehen,
   die Reihenfolge der Siloxaneinheiten beliebig ist, c, d und e unabhängig voneinander für 0 bis 100 stehen, wobei die Summe aus c, d und e mindestens 3 ist,
   f für eine ganze Zahl von 2 bis 8 steht,
   Z¹ für einen Rest der Formel VI

   -R²²-(CH₂CH₂O)_{g}(CH₂CH(CH₃)O)ₕ-R²³ (VI)

   steht, worin
   die Reihenfolge der Alkylenoxideinheiten beliebig ist und g und h unabhängig voneinander für eine ganze Zahl von 0 bis 200 stehen, wobei die Summe aus g und h > O ist,
   R²² für einen C₁- bis C₈-Alkylenrest steht und
   R²³ für Wasserstoff oder einen C₁- bis C₈-Alkylrest steht.
und Mischungen davon.

Bevorzugt stehen in der Formel V.1 R¹⁷ und R¹⁸ unabhängig voneinander für einen C₂- bis C₄-Alkylenrest. Insbesondere stehen R¹⁷ und R¹⁸ unabhängig voneinander für einen C₂- bis C₃-Alkylenrest.

Vorzugsweise liegt das Molekulargewicht der Verbindung der Formel V.1 in einem Bereich von etwa 300 bis 100 000 liegt.

Vorzugsweise steht in der Formel V.1 a für eine ganze Zahl von 1 bis 20, wie z. B. 2 bis 10.

Bevorzugt liegt die Gesamtzahl der Alkylenoxideinheiten der Verbindung der Formel V.1, d. h. die Summe aus v und w, in einem Bereich von etwa 3 bis 200, bevorzugt 5 bis 180.

Bevorzugt sind die Endgruppen der Polysiloxane mit Wiederholungseinheiten der allgemeinen Formel V.1 ausgewählt unter (CH₃)₃SiO, H, C₁- bis C₈-Alkyl und Mischungen davon.

Geeignete alkoxylierte Siloxanamine der Formel V.1 sind z. B. in der WO-A-97/32917 beschrieben, auf die hier in vollem Umfang Bezug genommen wird. Kommerziell erhältliche Verbindungen sind z. B. die Silsoft®-Marken der Fa. Witco, z. B. Silsoft® A-843.

Bevorzugt steht in der Formel V.2 der Rest R¹⁹ für einen C₂- bis C₄-Alkylenrest.

Bevorzugt stehen in der Formel V.2 R²⁰ und R²¹ unabhängig voneinander für Wasserstoff oder C₁- bis C₄-Alkyl.

Vorzugsweise wird die Summe aus c, d und e so gewählt, dass das Molekulargewicht der Verbindung der Formel V.2 in einem Bereich von etwa 300 bis 100 000, bevorzugt 500 bis 50 000, liegt.

Bevorzugt liegt die Gesamtzahl der Alkylenoxideinheiten des Restes der Formel VI, d. h. die Summe aus g und h, in einem Bereich von etwa 3 bis 200, bevorzugt 5 bis 80.

Bevorzugt steht in der Formel VI der Rest R²² für C₂- bis C₄-Alkyl.

Bevorzugt steht in der Formel VI der Rest R²³ für Wasserstoff oder C₁- bis C₄-Alkyl.

Eine geeignete Verbindung der Formel V.2 ist z. B. Silsoft® A-858 der Fa. Witco.

Weiterhin bevorzugt umfasst die Komponente VA) wenigstens ein pyrrolidongruppenhaltiges Polymer mit mindestens zwei freien primären, sekundären und/oder tertiären Aminogruppen pro Molekül. Vorzugsweise handelt es sich um ein pyrrolidongruppenhaltiges Polymer, wie zuvor als Komponente PB) beschrieben.

Als Neutralisationsmittel für die Polymere PA) können die zuvor genannten Verbindungen VA) jeweils einzeln oder in Form von Gemischen eingesetzt werden. Zur Neutralisation der Polymere PA) können auch Mischungen mehrerer Basen eingesetzt werden, die mindestens eine Verbindung VA) sowie mindestens eine weitere Base enthalten. Geeignete weitere Basen für die Neutralisation der Polymere sind Alkalimetallbasen, wie Natronlauge, Kalilauge, Natriumcarbonat, Kaliumcarbonat, Natriumhydrogencarbonat, Kaliumhydrogencarbonat und Erdalkalimetallbasen, wie Kalziumhydroxid, Kalziumoxid, Magnesiumhydroxid oder Magnesiumcarbonat sowie Ammoniak und Amine, die kein Gegenion B) ausbilden. Vorzugsweise werden zur Neutralisation der Polymere mit anionogenen Gruppen Gemische eingesetzt, die wenigstens ein Amin der allgemeinen Formel I und ein Alkalimetallhydroxid, bevorzugt Kaliumhydroxid, enthalten. Vorzugsweise werden zur Neutralisation der Polyurethane mit anionogenen Gruppen weiterhin Gemische eingesetzt, die wenigstens ein Amin der allgemeinen Formel I und einen Aminoalkohol, bevorzugt 2-Amino-2-methyl-1-propanol, enthalten.

Wird als Polymer ein Polyurethan eingesetzt, das zusätzlich Amingruppen als kationogene Gruppen aufweist, so können diese gewünschtenfalls durch Neutralisation mit einer Säure oder durch Quaternisierung teilweise oder vollständig in die entsprechenden kationischen Gruppen überführt werden.

Nach einer bevorzugten Ausführungsform setzt man zur Neutralisation eine Carbonsäure der Formel R²⁷-COOH ein, worin R²⁷ für einen C8- bis C₄₀-Alkylrest oder einen C₈- bis C₄₀-Alkenylrest steht. Bevorzugt wird eine Carbonsäure eingesetzt, die ausgewählt ist unter Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure, Melissinsäure, Tuberkulostearinsäure, Palmitoleinsäure, Oleinsäure, Linolsäure, Linolensäure, Eläostearinsäure und Mischungen davon. Geeignete Säuren zur Neutralisation sind auch anorganischen Säuren, wie Phosphorsäure, und Carbonsäuren, wie Milchsäure, und Mischungen der zuvor genannten Säuren.

Gewünschtenfalls können kationogene Gruppen auch teilweise oder vollständig quaternisiert werden. Die Quaternisierung kann z. B. mit Alkylierungsmitteln, wie C₁- bis C₄-Alkylhalogeniden oder Sulfaten erfolgen. Bevorzugte Alkylierungsmittel sind Ethylchlorid, Ethylbromid, Methylchlorid, Methylbromid, Dimethylsulfat und Diethylsulfat.

Nach einer weiteren bevorzugten Ausführungsform erfolgt die Herstellung der erfindungsgemäßen polymeren Salze durch Umsetzung wenigstens eines der zuvor genannten Polymere PB) mit einem Neutralisationsmittel, das wenigstens zwei- oder mehrwertige anorganische Säure und/oder eine Verbindung VB) mit mindestens zwei freien Säuregruppen pro Molekül aufweist.

Bevorzugt handelt es sich bei der Verbindung VB) um eine geradkettige oder verzweigte C₆- bis C₄₀-Di- oder Polycarbonsäure. Diese kann gewünschtenfalls eine oder mehrere zusätzliche funktionelle Gruppen, wie z. B. Hydroxylgruppen, aufweisen. Geeignete Di- und Polycarbonsäuren sind z. B. Adipinsäure, Azelainsäure, Dodecandisäure, Korksäure, Pimelinsäure, Sebacinsäure, Tetradecandisäure, Citronensäure etc.

Weiterhin bevorzugt handelt es sich bei der Verbindung VB) um ein pyrrolidongruppenhaltiges Polymer mit mindestens zwei-freien Carbonsäuregruppen pro Molekül. Vorzugsweise handelt es sich um ein pyrrolidongruppenhaltiges Polymer, wie zuvor als Komponente PA) beschrieben.

Geeignete zwei- oder mehrwertige anorganische Säuren sind z. B. H₃PO₄ oder H₂SO₄.

Als Neutralisationsmittel für die Polymere PB) können die zuvor genannten Verbindungen VB) jeweils einzeln oder in Form von Gemischen eingesetzt werden. Zur Neutralisation der Polymere PB) können auch Mischungen mehrerer Säuren eingesetzt werden, die mindestens eine Verbindung VB) sowie mindestens eine weitere Säure enthalten. Geeignete weitere Säuren für die Neutralisation der Polymere PB) sind die zuvor bei der Neutralisation kationogener Gruppen von Polyurethanen beschriebenen Carbonsäuren.

Bevorzugt beträgt der Anteil der Verbindung VA) oder VB) an dem polymeren Salz mindestens etwa 0,1 Gew.-%, insbesondere mindestens 0,5 Gew.-%.

Bevorzugt beträgt der Anteil der Verbindung VA) oder VB) an dem polymeren Salz höchstens etwa 50 Gew.-%, bevorzugt höchstens etwa 40 Gew.-%.

Bevorzugt sind polymere Salze, wie zuvor beschrieben, wobei das Verhältnis von
A) freien Säuregruppen des Polymers PA) zu freien Aminogruppen der Verbindung VA), oder
B) freien Aminogruppen des Polymers PB) zu freien Säuregruppen der Verbindung VB)
in einem Bereich von 1:0,005 bis 1:1, bevorzugt 1:0,01 bis 1:0,5, liegt.

Die Umsetzung der Polymere PA) und PB) mit dem Neutralisationsmittel kann vorzugsweise im Anschluss an die Herstellung der Polymere und im Allgemeinen im gleichen Reaktionsgefäß erfolgen. Gewünschtenfalls kann zur Herstellung der erfindungsgemäßen Salze auch ein separat hergestelltes oder kommerziell erhältliches Polymer eingesetzt werden. Geeignete Polyurethane werden z. B. in der DE-A-42 41 118, DE-A-42-25 045 und EP-A-0 619 111 beschrieben, auf die in vollem Umfang Bezug genommen wird. Geeignete Acrylatcopolymere werden z. B. in der DE-A-39 01 325 und der DE-A-43 14 305 beschrieben, auf die in vollem Umfang Bezug genommen wird. Geeignete pyrrolidongruppenhaltige Polymere werden z. B. in der DE-A-43 33 238 beschrieben, auf die in vollem Umfang Bezug genommen wird. Geeignete Lösungsmittel für die Umsetzung sind die zuvor bei der Herstellung der Polyurethane genannten.

Polyurethane, die sowohl kationogene als auch anionogene Gruppen aufweisen, können nacheinander einer Neutralisation mit wenigstens einer Säure, einer Neutralisation mit wenigstens einer Base und gewünschtenfalls zusätzlich einer Quaternisierung unterzogen werden. Die Reihenfolge der Neutralisationsschritte ist dabei im Allgemeinen beliebig.

Wird bei der Herstellung der polymeren Salze ein wassermischbares organisches Lösungsmittel eingesetzt, so kann dieses im Anschluss durch übliche, dem Fachmann bekannte Verfahren, z. B. durch Destillation bei vermindertem Druck, entfernt werden. Vor dem Abtrennen des Lösungsmittels kann dem polymeren Salz zusätzlich Wasser zugegeben werden. Nach Ersatz des Lösungsmittels durch Wasser erhält man eine Lösung oder Dispersion des polymeren Salzes, aus der, falls gewünscht, das polymere Salz in üblicher Weise gewonnen werden kann, z. B. durch Sprühtrocknung.

Der pH-Wert der wässrigen Lösungen oder Dispersionen kann durch Zugabe einer Säure oder Base eingestellt werden. Geeignete Säuren und Basen sind die zuvor als zusätzliche Neutralisierungsmittel genannten. Vorzugsweise liegt der pH-Wert für anionische polymere Salze im alkalischen Bereich, insbesondere > 7,5. Vorzugsweise liegt der pH-Wert für kationische polymere Salze im sauren Bereich, insbesondere bei 5,5 bis 6,5.

Die erfindungsgemäßen polymeren Salze sind wasserlöslich oder wasserdispergierbar. Sie bilden im Allgemeinen klare und klebfreie Filme und lassen sich mit Wasser sehr gut auswaschen. Vorteilhafterweise werden mit den erfindungsgemäßen polymeren Salzen auch Filme mit einer sehr guten Elastizität erhalten. Diese ist im Allgemeinen höher als die Elastizität, die üblicherweise bei aus dem Stand der Technik bekannten Polyurethanen mit kurzkettigen Gruppen erhalten wird. Haarbehandlungsmittel auf Basis dieser Salze verleihen dem Haar eine sehr gute Geschmeidigkeit.

Die erfindungsgemäßen polymeren Salze eignen sich besonders gut als oder in Haarfestigerpolymer(en), die dem Haar Flexibilität verleihen.

Die erfindungsgemäßen polymeren Salze sind als Hilfsmittel in der Kosmetik, bevorzugt als oder in Beschichtungsmittel(n) für keratinhaltige und keratinanaloge Oberflächen, wie Haar, Haut und Nägel, brauchbar. Sie eignen sich insbesondere für die Haarkosmetik, vorzugsweise als Festigerpolymer in Haarsprays, Schaumfestigern, Haarmousse, Haargel und Shampoos. Sie eignen sich weiterhin bevorzugt für eine Verwendung in der dekorativen Kosmetik, insbesondere in Mascara und Lidschatten. Die erfindungsgemäßen polymeren Salze sind weiterhin als Hilfsmittel in der Pharmazie, bevorzugt als oder in Beschichtungsmittel(n) oder Bindemittel(n) für feste Arzneiformen brauchbar. Die zuvor genannten polymeren Salze können auch in Cremes und als Tablettenüberzugmittel und Tablettenbindemittel verwendet werden. Sie eignen sich auch als Bindemittel und Klebemittel für kosmetische Produkte, z. B. bei der Herstellung stiftförmiger, kosmetischer Produkte, wie Deostifte, Schminkstifte etc. Die erfindungsgemäßen polymeren Salze eignen sich weiterhin vorzugsweise für die Verwendung als oder in Beschichtungsmittel(n) für die Textil-, Papier-, Druck-, Leder- und Klebstoffindustrie. Die erfindungsgemäßen polymeren Salze, die als Polymer PA) oder PB) und/oder als Verbindung VA) oder VB) wenigstens ein pyrrolidongruppenhaltiges Polymer enthalten, eignen sich vorzugsweise für die Verwendung in der Hautkosmetik, bevorzugt in Cremes.

Ein weitere Gegenstand der Erfindung ist ein kosmetisches oder pharmazeutisches Mittel, das wenigstens ein erfindungsgemäßes polymeres Salz enthält. Im Allgemeinen enthält das Mittel die polymeren Salze in einer Menge im Bereich von etwa 0,2 bis 30 Gew.-%, bevorzugt 0,5 bis 20 Gew.-%, bezogen auf das Gesamtgewicht des Mittels.

Die erfindungsgemäßen kosmetischen Mittel eignen sich insbesondere als Beschichtungsmittel für keratinhaltige und keratinanaloge Oberflächen (Haar, Haut und Nägel). Die in ihnen eingesetzten Verbindungen sind wasserlöslich oder wasserdispergierbar. Sind die in den erfindungsgemäßen Mitteln eingesetzten Verbindungen wasserdispergierbar, können sie in Form von wässrigen Mikrodispersionen mit Teilchendurchmessern von üblicherweise 1 bis 250 nm, bevorzugt 1 bis 150 nm, zur Anwendung gebracht werden. Die Feststoffgehalte der Präparate liegen dabei üblicherweise in einem Bereich von etwa 0,5 bis 20 Gew.-%, bevorzugt 1 bis 12 Gew.-%. Diese Mikrodispersionen benötigen in der Regel keine Emulgatoren oder Tenside zu ihrer Stabilisierung.

Bevorzugt können die erfindungsgemäßen Mittel in Form eines Haarbehandlungsmittels, wie Schaumfestiger, Haarmousse, Haargel, Shampoo und insbesondere in Form eines Haarsprays vorliegen. Zur Anwendung als Haarfestiger sind dabei Mittel bevorzugt, die polymere Salze enthalten, die wenigstens eine Glasübergangstemperatur Tg ≥ 0 °C, bevorzugt ≥ 10 °C, aufweisen. Der K-Wert dieser Polymere (gemessen nach E. Fikentscher, Cellulose-Chemie 13 (1932), S. 58-64) an einer 1 gew.-%igen Lösung in N-Methylpyrrolidon, liegt vorzugsweise in einem Bereich von 23 bis 90, insbesondere 25 bis 60. Weisen die erfindungsgemäßen Salze Siloxangruppen auf, so beträgt der Siloxangehalt dieser Polymere im Allgemeinen 0,05 bis 20 Gew.-%, bezogen auf das Gesamtgewicht der eingebauten Komponenten.

Vorzugsweise handelt es sich um Haarbehandlungsmittel. Diese liegen üblicherweise in Form einer wässrigen Dispersion oder in Form einer alkoholischen oder wässrig-alkoholischen Lösung vor. Beispiele geeigneter Alkohole sind Ethanol, Propanol, Isopropanol etc.

Weiter können die erfindungsgemäßen Haarbehandlungsmittel im Allgemeinen übliche kosmetische Hilfsstoffe enthalten, beispielsweise Weichmacher, wie Glycerin und Glykol; Emollienzien; Parfüms; Tenside; UV-Absorber; Farbstoffe; antistatische Mittel; Mittel zur Verbesserung der Kämmbarkeit; Konservierungsmittel; und Entschäumer.

Wenn die erfindungsgemäßen Mittel als Haarspray formuliert sind, enthalten sie eine ausreichende Menge eines Treibmittels, beispielsweise einen niedrigsiedenden Kohlenwasserstoff oder Ether, wie Propan, Butan, Isobutan oder Dimethylether. Als Treibmittel sind auch komprimierte Gase brauchbar, wie Stickstoff, Luft oder Kohlendioxid. Die Menge an Treibmittel kann dabei gering gehalten werden, um den VOC-Gehalt nicht unnötig zu erhöhen. Sie beträgt dann im Allgemeinen nicht mehr als 55 Gew.-%, bezogen auf das Gesamtgewicht des Mittels. Gewünschtenfalls sind aber auch höhere VOC-Gehalte von 85 Gew.-% und darüber möglich.

Die zuvor beschriebenen Polyurethane können auch in Kombination mit anderen Haarpolymeren in den Mitteln zur Anwendung kommen. Solche Polymere sind insbesondere:
- nicht-ionische, wasserlösliche bzw. wasserdispergierbare Polymere oder Oligomere, wie Polyvinylcaprolactam, z. B. Luviskol Plus (BASF), oder Polyvinylpyrrolidon und deren Copolymere, insbesondere mit Vinylestern, wie Vinylacetat, z. B. Luviskol VA 37 (BASF); Polyamide, z. B. auf Basis von Itaconsäure und aliphatischen Diaminen, wie sie z. B. in der DE-A-43 33 238 beschrieben sind;
- amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557; DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung Amersette® (AMERCHOL) im Handel erhältlich sind, und Copolymere aus Hydroxyethylmethacrylat, Methylmethacrylat, N,N-Dimethylaminoethylmethacrylat und Acrylsäure (Jordapon®);
- anionische Polymere, wie Vinylacetat/Crotonsäure-Copolymere, wie sie beispielsweise unter den Bezeichnungen Resyn® (NATIONAL STARCH), Luviset® (BASF) und Gafset® (GAF) im Handel sind, Vinylpyrrolidon/Vinylacrylat-Copolymere, erhältlich beispielsweise unter dem Warenzeichen Luviflex® (BASF). Ein bevorzugtes Polymer ist das unter der Bezeichnung Luviflex® VBM-35 (BASF) erhältliche Vinylpyrrolidon/Acrylat-Terpolymer. Acrylsäure/Ethylacrylat/N-tert.Butylacrylamid-Terpolymere, die beispielsweise unter der Bezeichnung Ultrahold® strong (BASF) vertrieben werden, sowie Luvimer® (BASF, Terpolymer aus t-Butylacrylat, Ethylacrylat und Methacrylsäure), Natriumsulfonat-haltige Amide oder Natriumsulfonat-haltige Polyester, oder
- kationische (quaternisierte) Polymere, z. B. kationische Polyacrylatcopolymere auf Basis von N-Vinyllactamen und deren Derivaten (N-Vinylpyrrolidon, N-Vinylcaprolactam etc.) sowie übliche kationische Haarconditionerpolymere, z. B. Luviquat® (Copolymer aus Vinylpyrrolidon und Vinylimidazoliummethochlorid), Luviquat® Hold (Copolymerisat aus quaternisiertem N-Vinylimidazol, N-Vinylpyrrolidon und N-Vinylcaprolactam), Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen), Polyquaternium-Typen (CTFA-Bezeichnungen) etc.;
- nichtionische, siloxanhaltige, wasserlösliche oder -dispergierbare Polymere, z. B. Polyethersiloxane, wie Tegopren® (Fa. Goldschmidt) oder Belsil® (Fa. Wacker).

Die erfindungsgemäßen Polymere können als Mischung mit einem amidgruppenhaltigen Haarpolymer eingesetzt. Dazu zählen z. B. die in der DE-A-42 25 045 beschriebenen Polyurethane, die zuvor beschriebenen Vinylpyrrolidon/Acrylat-Terpolymere und Acrylsäure/Ethylacrylat/N-tert.-Butylacrylamid-Terpolymere (z. B. Ultrahold®strong der BASF AG), die zuvor beschriebenen amidgruppenhaltigen amphoteren Polymere (z. B. Amphomer®) und insbesondere Copolymerisate, die einen Anteil an amidgruppenhaltigen Monomeren, wie N-Vinyllactamen, von mindestens 30 Gew.-% aufweisen (z. B. Luviskol®plus und Luviskol®VA37 der BASF AG).

Die anderen Haarpolymere sind vorzugsweise in Mengen bis zu 10 Gew.-%, bezogen auf das Gesamtgewicht des Mittels enthalten.

Ein bevorzugtes Haarbehandlungsmittel enthält:
a) 0,5 bis 20 Gew.-%, bevorzugt 1 bis 10 Gew.-%, mindestens eines in Wasser löslichen oder dispergierbaren, erfindungsgemäßen polymeren Salzes,
b) 50 bis 99,5 Gew.-%, bevorzugt 55 bis 99 Gew.-%, eines Lösungsmittels, ausgewählt unter Wasser und wassermischbares Lösungsmitteln, bevorzugt C₂- bis C₅-Alkoholen, insbesondere Ethanol, und Mischungen davon,
c) 0 bis 70 Gew.-%, bevorzugt 0,1 bis 50 Gew.-%, eines Treibmittels, vorzugsweise ausgewählt unter Dimethylether und Alkanen, wie z. B. Propan/Butan-Gemischen,
d) 0 bis 10 Gew.-%, bevorzugt 0,1 bis 10 Gew.-%, mindestens eines von a) verschiedenen, in Wasser löslichen oder dispergierbaren Haarpolymers,
e) 0 bis 0,5 Gew.-%, bevorzugt 0,001 bis 2 Gew.-%, mindestens einer wasserlöslichen oder wasserdispergierbaren Siliconverbindung,
sowie übliche Zusatzstoffe.

Das erfindungsgemäße Mittel kann als Komponente d) mindestens ein anderes, in Wasser lösliches oder dispergierbares Haarpolymer enthalten. Der Anteil dieser Komponente beträgt dann im Allgemeinen etwa 0,1 bis 10 Gew.-%, bezogen auf das Gesamtgewicht des Mittels. Bevorzugt können dabei wasserlösliche oder wasserdispergierbare Polyurethane eingesetzt werden, die Siloxangruppen einpolymerisiert enthalten.

Das erfindungsgemäße Mittel kann als Komponente e) mindestens ein nichtionisches, siloxanhaltiges, wasserlösliches oder -dispergierbares Polymer, insbesondere ausgewählt unter den zuvor beschriebenen Polyethersiloxanen, enthalten. Der Anteil dieser Komponente beträgt dann im Allgemeinen etwa 0,001 bis 2 Gew.-%, bezogen auf das Gesamtgewicht des Mittels.

Das erfindungsgemäße Mittel kann als zusätzliche Komponente mindestens ein wasserunlösliches Silicon, insbesondere ein Polydimethylsiloxan, z. B. die Abil®-Typen der Fa. Goldschmidt, enthalten. Der Anteil dieser Komponente beträgt dann im Allgemeinen etwa 0,0001 bis 0,2 Gew.-%, bevorzugt 0,001 bis 0,1 Gew.-%, bezogen auf das Gesamtgewicht des Mittels.

Das erfindungsgemäße Mittel kann zusätzlich gegebenenfalls einen Entschäumer, z. B. auf Silicon-Basis, enthalten. Die Menge des Entschäumers beträgt im Allgemeinen bis zu etwa 0,001 Gew.-%, bezogen auf die Gesamtmenge des Mittels.

Das erfindungsgemäße Mittel enthält vorzugsweise zusätzlich zu den zuvor genannten Komponenten:
f) 0 bis 40 Gew.-%, bevorzugt 0,1 bis 35 Gew.-%, wenigstens eines Tensids,
g) 0 bis 5 Gew.-%, bevorzugt 0,05 bis 4 Gew.-%, wenigstens eines Farbstoffes und/oder UV-Absorbers,
h) 0 bis 3 Gew.-%, bevorzugt 0,05 bis 2,5 Gew.-%, wenigstens eines Salzes,
i) 0 bis 3 Gew.-%, bevorzugt 0,05 bis 2,5 Gew.-%, wenigstens eines Verdickers,
sowie gegebenenfalls weitere übliche Zusatzstoffe. Diese liegen dann im Allgemeinen jeweils in einer Menge von etwa 0 bis 0,2 Gew.-%, bevorzugt 0,001 bis 0,2 Gew.-%, bezogen auf das Gesamtgewicht des Mittels, vor.

Die erfindungsgemäßen Mittel besitzen den Vorteil, dass sie einerseits den Haaren die gewünschte Festigkeit verleihen und die Polymere leicht auswaschbar (redispergierbar) sind und das Haar andererseits elastisch bleibt.

Vorteilhafterweise eignen sich die erfindungsgemäßen Salze auch als Komponente in Haarbehandlungsmitteln, die zusätzlich wenigstens ein weiteres herkömmliches Haarpolymer enthalten. Auch mit diesen Mischungen werden im Allgemeinen bessere Flexibilitäten erzielt als bei den entsprechenden Polymeren oder Mischungen, die die erfindungsgemäßen Salze nicht enthalten. Die erfindungsgemäßen Salze eignen sich somit auch zur Verbesserung der Elastizität herkömmlicher Haarfestigungsmittel. Diese verleihen den Haaren dann im Allgemeinen eine sehr gute Flexibilität und Geschmeidigkeit.

Die Erfindung wird anhand der folgenden, nicht einschränkenden Beispiele näher erläutert.

### Beispiele

Die Beurteilung der anwendungstechnischen Eigenschaften erfolgte durch Notenvergabe von unabhängigen Fachleuten. Die getesteten Eigenschaften und die Notenskala sind in Tabelle 1 wiedergegeben. Für die Beurteilung von Elastizität, Zugfestigkeit und Griff wurden jeweils 20 gew.-%ige ethanolische Lösungen der polymeren Salze als 500 µm-Film auf Polyethylenfolie gerakelt und getrocknet. Für die Beurteilung von Klebrigkeit, Auswaschbarkeit und Klarheit wurden jeweils 5 gew.-%ige ethanolische Lösungen auf eine Glasplatte aufgetragen und getrocknet.

**Tabelle 1**

| | Note | | | |
|---|---|---|---|---|
| | 1 | 2 | 3 | 4 |
| Elastizität | sehr weich | mäßig weich | hart | spröde |
| Zugfestigkeit | fest | mäßig fest | schlecht | verfließend |
| Griff | geschmeidigglatt | mäßig glatt | hemmend | rauh hemmend |
| Klebrigkeit | nicht klebrig | befriedigend | leicht klebrig | klebrig |
| Auswaschbarkeit | sehr gut löslich | befriedigend | schlecht löslich | nicht löslich |
| Klarheit | klar | befriedigend | trüb | milchig trüb |

### Polymer P1:

### Herstellung eines Polyurethans mit freien Carbonsäuren

In einem Rührapparat, der mit Rührer, Tropftrichter, Thermometer, Rückflusskühler und einer Vorrichtung für das Arbeiten unter Stickstoff ausgestattet war, wurden 1 000 g (1 mol) eines Polyesterdiols (Mₙ = 1 000 g/mol, hergestellt aus Isophthalsäure, Adipinsäure und 1,6-Hexandiol), 124,8 g (1,2 mol) Neopentylglykol, 361,8 g (2,7 mol) Dimethylolpropansäure und 0,5 g Tetrabutylorthotitanat in 350 g Methylethylketon unter Erhitzen auf eine Temperatur von etwa 50 °C und unter Rühren gelöst. Anschließend wurden unter Rühren 1111 g (5 mol) Isophorondiisocyanat zugetropft, wobei die Reaktionstemperatur anstieg. Unter Rückfluss wurde das Reaktionsgemisch dann solange gerührt, bis der Isocyanatgruppengehalt des Gemisches praktisch konstant blieb, und anschließend unter Rühren auf Raumtemperatur abgekühlt. Dann gab man zur Umsetzung der freien Isocyanatgruppen 13,4 g (0,1 mol) 2-Amino-2-methyl-1-propanol in Form einer wässrigen Lösung bei einer Temperatur von etwa 40 °C zu dem Gemisch. Abschließend wurde das so erhaltene Polyurethan dann mit 2,7 mol eines Amingemischs aus 2-Amino-2-methyl-1-propanol und einem Amin gemäß Tabelle 2 zu 100 % neutralisiert. Dabei wurde das Mengenverhältnis von 2-Amino-2-methyl-1-propanol zu Amin so gewählt, dass die in Tabelle 4 angegebenen Mengenverhältnisse von Polyurethan zu Amin erzielt wurden. Die anwendungstechnischen Eigenschaften der so erhaltenen polymeren Salze sind ebenfalls in Tabelle 4 wiedergegeben. Nach Abdestillieren des Lösungsmittels im Vakuum bei 40 °C erhält man eine wässrige (Mikro)dispersion. Pulverförmige Produkte können durch Sprühtrocknen erhalten werden.

### Polymer P2:

### Herstellung eines Polyacrylats mit freien Carbonsäuregruppen

In einer Rührapparatur, die mit Rückflusskühler, 4 separaten Zulaufvorrichtungen und einer Vorrichtung für das Arbeiten unter Stickstoff ausgestattet war, wurden 32,0 g von Zulauf 1 und 10,0 g von Zulauf 2 vorgelegt und die Mischung auf 80 °C aufgeheizt. Nach dem Anpolymerisieren, erkennbar an einer beginnenden Viskositätserhöhung, wurde der Rest von Zulauf 1 innerhalb von 3 Stunden und der Rest von Zulauf 2 innerhalb von 4 Stunden zugegeben, wobei die Innentemperatur auf etwa 80 °C gehalten wurde. Anschließend ließ man 2 Stunden bei dieser Temperatur nachpolymerisieren. Dann wurde Zulauf 3 innerhalb von 1 Stunde zugegeben, wobei die Innentemperatur weiterhin auf 80 °C gehalten wurde. Nach dem Ende der Zugabe wurde noch 5 Stunden bei dieser Temperatur nachpolymerisiert. Nach dem Abkühlen wurde das Reaktionsgemisch mit Zulauf 4 verdünnt. Abschließend wurde das so erhaltene Polymer dann mit 0,85 mol eines Amingemischs aus 2-Amino-2-methyl-1-propanol und einem Amin gemäß Tabelle 2 zu 100 % neutralisiert. Dabei wurde das Mengenverhältnis von 2-Amino-2-methyl-1-propanol zu Amin so gewählt, dass die in Tabelle 4 angegebenen Mengenverhältnisse von Polymer zu Amin erzielt wurden. Die anwendungstechnischen Eigenschaften der so erhaltenen polymeren Salze sind ebenfalls in Tabelle 4 wiedergegeben.

| | | |
|---|---|---|
| Zulauf 1: | n-Butylacrylat tert.-Butylacrylat Methacrylsäure | 120,0 g |
| | | 105,0 g |
| | | 75,0 g |
| Zulauf 2: | Ethanol tert.-Butylperpivalat | 100,0 g |
| | | 0,9 g |
| Zulauf 3: | Ethanol tert.-Butylperpivalat | 150,0 g |
| | | 1,6 g |
| Zulauf 4: | Ethanol | 200,0 g |

### Polymer P3:

### Herstellung eines Polyurethans mit freien Aminogruppen

In einem Rührapparat, der mit Rührer, Tropftrichter, Thermometer, Rückflusskühler und einer Vorrichtung für das Arbeiten unter Stickstoff ausgestattet war, wurden 500 g (0,5 mol) eines Polytetrahydrofurans (Mₙ = 1 000 g/mol), 124,8 g (1,2 mol) Neopentylglykol, 381,3 g (3,1 mol) N-Methyldiethanolamin und 0,3 g Tetrabutylorthotitanat in 350 g Methylethylketon unter Erhitzen auf eine Temperatur von etwa 50 °C und unter Rühren gelöst. Anschließend wurden unter Rühren 1111 g (5 mol) Isophorondiisocyanat zugetropft, wobei die Reaktionstemperatur anstieg. Unter Rückfluss wurde das Reaktionsgemisch dann solange gerührt, bis der Isocyanatgruppengehalt des Gemisches praktisch konstant blieb, und anschließend unter Rühren auf Raumtemperatur abgekühlt. Dann gab man zur Umsetzung der freien Isocyanatgruppen 40,2 g (0,3 mol) 2-Amino-2-methyl-1-propanol in Form einer wässrigen Lösung bei einer Temperatur von etwa 40 °C zu dem Gemisch. Abschließend wurde das so erhaltene Polyurethan dann mit 2,7 mol eines Säuregemisches aus Milchsäure und einer Säure gemäß Tabelle 3 zu 100 % neutralisiert. Dabei wurde das Mengenverhältnis von Milchsäure zu Säure gemäß Tabelle 3 so gewählt, dass die in Tabelle 4 angegebenen Mengenverhältnisse von Polyurethan zu Amin erzielt wurden. Die anwendungstechnischen Eigenschaften der so erhaltenen polymeren Salze sind ebenfalls in Tabelle 4 wiedergegeben. Nach Abdestillieren des Lösungsmittels im Vakuum bei 40 °C erhält man eine wässrige (Mikro)dispersion. Pulverförmige Produkte können durch Sprühtrocknen erhalten werden.

**Tabelle 2**

| Zur Neutralisation der Polymere eingesetzte mehrwertige Amine | |
|---|---|
| Amin | |
| A1 | N-Methyldipropylentriamin |
| A2 | n-Talg-propylendiamin (7 EO-Einheiten)¹⁾ |
| A3 | Diaminopolyether/ethoxyliertes Oleylamin (2 EO-Einheiten)²⁾, Molverhältnis 1:1 |
| A4 | pyrrolidongruppenhaltiges Polyamid (Itaconsäure:Hexamethylendiamin, Molverhältnis 5:6) |
| A5 | Diaminopolyethersiloxan³⁾ |

| | |
|---|---|
| 1) Dinoramox® S7, Fa. Ceca | |
| 2) Noramox® 02, Fa. Ceca | |
| 3) Silsoft® A-843, Fa. Witco | |

**Tabelle 3**

| Zur Neutralisation der Polymere eingesetzte mehrwertige Säuren | |
|---|---|
| Säure | |
| S1 | pyrrolidongruppenhaltiges Polyamid (Itaconsäure:Hexamethylendiamin, Molverhältnis 6:5) |
| S2 | H₃PO₄ |

**Tabelle 4**

| Bsp. Nr. | Polymer:Polyamin Gewicht-Verhältnis | Elastizität | Zugfestigkeit | Griff | Klebrigkeit | Auswaschbarkeit | Klarheit |
|---|---|---|---|---|---|---|---|
| V1 | Polyurethan P1 mit AMP⁴) neutral. | 3 | 2 | 2 | 1-2 | 1-2 | 1 |
| V2 | anion. Polyacrylat P2 mit AMP neutral. | 2-3 | 2-3 | 2 | 3 | 1 | 1 |
| V3 | kation. Polyurethan P3 mit MIS⁵⁾ neutral. | 2-3 | 2 | 2 | 3 | 1 | 1 |
| 1 | P1 : A1 | 1 | 2 | 2 | 1-2 | 2 | 1-2 |
| | 95 : 5 | | | | | | |
| 2 | P1 : A2 | 2-3 | 1-2 | 1-2 | 1-2 | 1-2 | 1 |
| | 95 : 5 | | | | | | |
| 3 | P1 : A2 | 1-2 | 2 | 1-2 | 2 | 1 | 1 |
| | 90 : 10 | | | | | | |
| 4 | P1 : A2 | 1 | 3-4 | 2 | 3 | 1 | 1 |
| | 80 : 20 | | | | | | |
| 5 | P1 : A3 | 2 | 2-3 | 2 | 2 | 1 | 1 |
| | 90 : 10 | | | | | | |
| 6 | P1 : A4 | 2 | 2 | 2 | 2 | 1-2 | 1-2 |
| | 90 : 10 | | | | | | |
| 7 | P1 : A5 | 1-2 | 2-3 | 1 | 1-2 | 1-2 | 1-2 |
| | 90 : 10 | | | | | | |
| 8 | P2 : A1 | 1-2 | 2 | 2 | 3 | 1 | 1 |
| | 90 : 10 | | | | | | |
| 9 | P2 : A2 | 2 | 3 | 1-2 | 2 | 1 | 1 |
| | 90 : 10 | | | | | | |
| 10 | P2 : A3 | 2-3 | 3 | 1-2 | 2 | 1-2 | 1 |
| | 90 : 10 | | | | | | |
| 11 | P2 : A4 | 2 | 2 | 2-3 | 2-3 | 1 | 2 |
| | 90 : 10 | | | | | | |
| 12 | P2 : A5 | 1-2 | 3 | 1 | 2 | 1 | 1 |
| | 90 : 10 | | | | | | |
| 13 | P3 : S1 | 2 | 2 | 2 | 3 | 1-2 | 1-2 |
| | 90 : 10 | | | | | | |
| 14 | P3 : S2 | 2 | 1-2 | 1-2 | 2-3 | 2 | 1 |
| | 95 : 5 | | | | | | |

### Anwendungstechnische Beispiele

### Beispiele 15 bis 28

Aerosol-Haarspray-Formulierungen mit einem VOC-Gehalt von 97 Gew.-%:

| | | |
|---|---|---|
| Polymeres Salz gemäß Beisp. 1-14 | 3,00 | Gew.-% |
| Ethanol | 57,00 | Gew.-% |
| Dimethylether | 34,96 | Gew.-% |
| Parfüm, Zusatzstoffe | q.s. | |

### Beispiele 29 bis 42

Kompakte Aerosol-Haarspray-Formulierungen mit einem VOC-Gehalt von 80 Gew.-%:

| | | |
|---|---|---|
| Polymeres Salz gemäß Beisp. 1-14 | 5,00 | Gew.-% |
| Ethanol | 40,00 | Gew.-% |
| Dimethylether | 34,96 | Gew.-% |
| Wasser | 15,00 | Gew.-% |
| Parfüm, Zusatzstoffe | q.s. | |

### Beispiele 43 bis 56

Haarspray-Formulierungen mit einem VOC-Gehalt von 55 Gew.-%:

| | | |
|---|---|---|
| Polymeres Salz gemäß Beisp. 1-14 | 3,00 | Gew.-% |
| Ethanol | 20,00 | Gew.-% |
| Wasser | 42,00 | Gew.-% |
| Dimethylether | 34,96 | Gew.-% |
| Parfüm, Zusatzstoffe | q.s. | |

### Beispiele 57 bis 70

Pump-Haarspray:

| | | |
|---|---|---|
| Polyurethan gemäß Beisp. 1-14 | 5,00 | Gew.-% |
| Ethanol | 54,96 | Gew.-% |
| Wasser | 40,00 | Gew.-% |
| Parfüm, Zusatzstoffe | q.s. | |

### Beispiele 71 bis 84

| | |
|---|---|
| Schaum-Conditioner | :[Gew.-%] |
| Polymer 1-14 (25%ige wässrige Lösung) | |
| | 20,00 |
| Cremophor® A25⁴⁾ | 0,20 |
| Comperlan® KD⁵⁾ | 0,10 |
| Wasser | 69,70 |
| Propan/Butan | 9,96 |
| Parfüm, Konservierungsmittel q.s. | |

| | |
|---|---|
| 4) CTFA-Name: Ceteareth 25, Fa. BASF AG, Umsetzungsprodukt aus Fettalkohol und Ethylenoxid | |
| 5) CTFA-Name: Cocamide DEA, Fa. Henkel, Kokosfettsäureamid | |

Zur Herstellung der Schaum-Conditioner werden die Komponenten eingewogen und unter Rühren gelöst. Anschließend werden sie in einen Spender abgefüllt und das Treibgas zugesetzt.

### Beispiele 85 bis 98

| Conditioner-Shampoo: | | [Gew. -%] |
|---|---|---|
| A) | Texapon® NSO 28%ig⁶⁾ | 50,00 |
| | Comperlan® KD | 1,00 |
| | Polymer 1-14 (25%ige wässrige Lösung) | 20,00 |
| | Parfümöl | q.s. |
| B) | Wasser | 27,5 |
| | Natriumchlorid | 1,5 |
| | Konservierungsmittel | q.s. |

| | | |
|---|---|---|
| ⁶⁾ Natriumlaurylsulfat, Fa. Henkel | | |

zur Herstellung der Conditioner-Shampoos werden die Komponenten A) und B) getrennt eingewogen und unter Mischen gelöst. Dann wird Phase B) langsam unter Rühren zu Phase A) gegeben.

### Beispiele für Anwendungen in der Hautkosmetik

### Beispiele 99 bis 112

### O/W-Cremes

| O/W-Cremes | | |
|---|---|---|
| Ölphase: | Gew.-% | CTFA-Name: |
| Cremophor® A6 (BASF AG) | 3,5 | Ceteareth-6 (Stearylalkohol-Ethoxylat) |
| Cremophor® A25 (BASF AG) | 3,5 | Ceteareth-25 (Fettalkohol-Ethoxylat) |
| Glycerinmonostearat s.e. | 2,5 | Glycerylstearate |
| Paraffinöl | 7,5 | |
| Cetylalkohol | 2,5 | |
| Luvitol® EHO (BASF AG) | 3,2 | Cetearyloctanoat |
| Vitamin-E-acetate | 1,0 | Tocopherylacetat |
| Nip-Nip®, Nipa Laboratories Ltd., USA | 0,1 | Methyl- und Propyl-4-hydroxybenzoate (7:3) |

| Wasserphase: | Gew.-% | CTFA-Name: |
|---|---|---|
| Polymer 1-14 | 1,5 | |
| Wasser | 73,6 | |
| 1,2-Propylenglykol | 1,0 | Propylenglykol |
| Germall II, Sutton Laboratories Inc., USA | 0,1 | Imidazolidinylharnstoff |

Zur Herstellung der Cremes werden die Komponenten für Öl- und Wasserphase getrennt eingewogen und bei 80 °C homogenisiert. Dann gibt man die Wasserphase langsam unter Rühren zu der Ölphase. Anschließend lässt man unter Rühren auf Raumtemperatur abkühlen.

### Beispiele 113 bis 126

### O/W-Lotions

| Ölphase: | Gew.-% | CTFA-Name: |
|---|---|---|
| Cremophor® A6 (BASF AG) | 2,0 | Ceteareth-6 (Stearylalkohol-Ethoxylat) |
| Cremophor® A25 (BASF AG) | 2,0 | Ceteareth-25 (Fettalkohol-Ethoxylat) |
| Glycerinmonostearat s.e. | 6,0 | Glycerylstearate |
| Paraffinöl | 0,9 | Paraffinöl |
| Tegiloxan® 100 | 0,1 | Dimethicone (Polydimethylsiloxan) |
| Cetylalkohol | 1,5 | Cetylalkohol |
| Luvitol® EHO (BASF AG) | 12,0 | Cetearyloctanoat |
| Vitamin-E-acetate | 0,4 | Tocopherylacetat |
| Nip-Nip®, Nipa Laboratories Ltd., USA | 0,1 | Methyl- und Propyl-4-hydroxybenzoate (7:3) |

| Wasserphase: | Gew.-% | CTFA-Name: |
|---|---|---|
| Polymer 1-14 | 0,5 | |
| Wasser | 73,4 | |
| 1,2-Propylenglykol | 1,0 | Propylenglykol |
| Germall II, Sutton Laboratories Inc., USA | 0,1 | Imidazolidinylharnstoff |

Zur Herstellung der O/W-Lotionen werden die Komponenten für Ölund Wasserphase getrennt eingewogen und bei 80 °C homogenisiert. Dann gibt man die Wasserphase langsam unter Rühren zu der Ölphase. Anschließend lässt man unter Rühren auf Raumtemperatur abkühlen.

## Patentansprüche

1. Wasserlösliches oder wasserdispergierbares polymeres Salz aus :
A) wenigstens einem Polymer PA) mit freien Säuregruppen und einem Neutralisationsmittel, das wenigstens eine Verbindung VA) mit mindestens zwei freien Aminogruppen pro Molekül umfasst, oder
B) wenigstens einem Polymer PB) mit freien Aminogruppen und einem Neutralisationsmittel, das wenigstens eine zweioder mehrwertige anorganische Säure und/oder wenigstens eine Verbindung VB) mit mindestens zwei freien Säuregruppen pro Molekül umfasst,
wobei die Verbindungen VA) und VB) zusätzlich wenigstens eine hydrophile Gruppe aufweisen, die ausgewählt ist unter weiteren ionogenen und/oder ionischen Gruppen, zweiwertigen Resten von Polyethern, zweiwertigen Resten von pyrrolidongruppenhaltigen Polymeren und Kombinationen davon.

2. Polymeres Salz nach Anspruch 1, wobei die Komponente PA) oder PB) mindestens ein Polyurethan umfasst, welches
a) mindestens ein Polymerisat mit wenigstens zwei aktiven Wasserstoffatomen pro Molekül,
b) mindestens eine Verbindung, die zwei aktive Wasserstoffatome und mindestens eine ionogene und/oder ionische Gruppe pro Molekül aufweist,
c) gegebenenfalls mindestens eine Verbindung mit einem Molekulargewicht im Bereich von 56 bis 500, die zwei aktive Wasserstoffatome pro Molekül enthält, und
d) mindestens ein Diisocyanat
eingebaut enthält.

3. Polymeres Salz nach einem der Ansprüche 1 oder 2, wobei die Komponente PA) mindestens ein Polymer umfasst, welches
e) wenigstens eine α,β-ethylenisch ungesättigte Mono- und/oder Dicarbonsäure,
f) wenigstens ein α,β-ethylenisch ungesättigtes Monomer, das ausgewählt ist unter Estern α,β-ethylenisch ungesättigter Mono- und/oder Dicarbonsäuren mit geradkettigen und/oder verzweigten C₁- bis C₆-Alkanolen, Amiden α,β-ethylenisch ungesättigter Mono- und/oder Dicarbonsäuren mit Mono- und Dialkylaminen mit geradkettigen und/oder verzweigten C₁bis C₆-Alkylresten und Mischungen davon,
g) gegebenenfalls wenigstens ein weiteres, von e) und f) verschiedenes Monomer mit mindestens einer α,β-ethylenisch ungesättigten Doppelbindung,
einpolymerisiert enthält.

4. Polymeres Salz nach einem der vorhergehenden Ansprüche, wobei die Komponente PA) oder PB) mindestens ein pyrrolidongruppenhaltiges Polymer umfasst, erhältlich durch Umsetzung eines Monomerengemisches, welches
h) Itaconsäure und/oder ein Derivat davon, und
i) wenigstens ein Diamin der allgemeinen Formel I
H₂N-A-NHR¹ (I)
worin
R¹ für Wasserstoff oder C₁- bis C₄-Alkyl steht,
A für einen C₂- bis C₂₀-Alkylenrest steht, der durch wenigstens eine oder mehrere nicht benachbarte, gleiche oder verschiedene Gruppen -NR²- unterbrochen sein kann, wobei R² für Wasserstoff, C₁- bis C₄-Alkyl, C₅bis C₈-Cycloalkyl, Phenyl oder Phenyl-C₁-C₄-alkyl steht,
enthält.

5. Polymeres Salz nach einem der vorhergehenden Ansprüche, wobei die Komponente VA) wenigstens ein Polyamin der Formel II 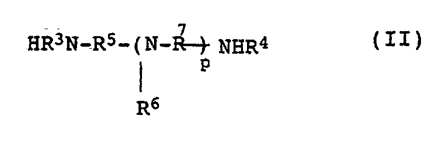 umfasst, worin
p für eine ganze Zahl von 0 bis 4 steht,
R³ und R⁴ unabhängig voneinander für Wasserstoff, C₁- bis C₄₀-Alkyl oder C₆- bis C₄₀-Alkenyl stehen, wobei die Alkyl- und Alkenylreste wenigstens eine ionogene und/oder ionische Gruppe tragen können, die ausgewählt ist unter -COOY, -SO₃Y und -PO₃Y, wobei Y für H, Li, Na, K oder Ammonium steht, wobei für p = 0 wenigstens einer der Reste R³ oder R⁴ für einen C₁- bis C₄₀-Alkyl- oder C₆- bis C₄₀-Alkenylrest steht, der wenigstens eine ionogene und/oder ionische Gruppe trägt,
R⁵ und R⁷ für einen C₂- bis C₆-Alkylenrest stehen, wobei für p > 1 die Reste R⁷ unabhängig unter C₂- bis C₆-Alkylenresten ausgewählt sind,
R⁶ für C₁- bis C₆-Alkyl, C₅- bis C₈-Cycloalkyl, Phenyl oder Phenyl-C₁-C₄-alkyl steht, wobei für p > 1 die Reste R⁶ unabhängig unter diesen Bedeutungen ausgewählt sind.

6. Polymeres Salz nach einem der vorhergehenden Ansprüche, wobei die Komponente VA) wenigstens einen Diaminopolyether der Formel III
HR⁸N-R¹⁰-O-(CH₂CH₂O)_{q}(C₃H₆O)ᵣ-R¹¹-NHR⁹ (III)
umfasst, worin
R⁸ und R⁹ unabhängig voneinander für Wasserstoff, C₁- bis C₄₀-Alkyl oder C₆- bis C₄₀-Alkenyl stehen, wobei die Alkyl- und Alkenylreste gegebenenfalls wenigstens eine ionogene und/oder ionische Gruppe tragen können, die ausgewählt ist unter -COOY, -SO₃Y und -PO₃Y, wobei Y für H, Li, Na, K oder Ammonium steht,
R¹⁰ und R¹¹ unabhängig voneinander für einen C₂- bis C₆-Alkylenrest stehen,
die Reihenfolge der Alkylenoxideinheiten beliebig ist und q und r unabhängig voneinander für eine ganze Zahl von 0 bis 100 stehen, wobei die Summe aus q und r in einem Bereich von 5 bis 100 liegt.

7. Polymeres Salz nach einem der vorhergehenden Ansprüche, wobei die Komponente VA) wenigstens ein alkoxiliertes Amin der Formel IV
R¹²R¹⁴N-R¹⁶-NR¹³R¹⁵ (IV)
umfasst, worin
R¹² und R¹³ unabhängig voneinander für C₁- bis C₄₀-Alkyl, C6-bis C₄₀-Alkenyl, Hydroxyalkyl oder einen Rest der Formel -(CH₂CH₂O)ₘ(C₃H₆O)ₙ-H stehen, wobei die Reihenfolge der Alkylenoxideinheiten beliebig ist und m und n unabhängig voneinander für eine ganze Zahl von 0 bis 30 stehen, wobei die Summe aus m und n in einem Bereich von 1 bis 30 liegt,
R¹⁴ und R¹⁵ unabhängig voneinander ausgewählt sind unter den für R¹² und R¹³ angegebenen Bedeutungen und C₂- bis C₆-Alkylresten mit einer ionogenen oder ionischen Gruppe, die ausgewählt ist unter -COOY, -SO₃Y und -PO₃Y, wobei Y für H, Li, Na, K oder Ammonium steht, und
R¹⁶ für einen C₂- bis C₆-Alkylenrest steht.

8. Polymeres Salz nach einem der vorhergehenden Ansprüche, wobei die Komponente VA) wenigstens ein Diaminopolyethersiloxan der Formel V umfasst, das ausgewählt ist unter
- Polysiloxanen mit Wiederholungseinheiten der allgemeinen Formel V.1 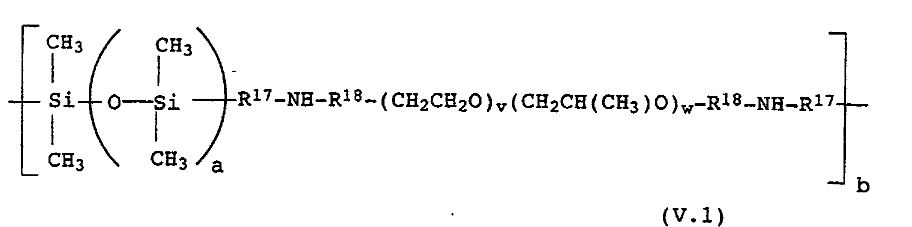
worin
a für eine ganze Zahl von 0 bis 100 steht,
b für eine ganze Zahl von 1 bis 8 steht,
R¹⁷ und R¹⁸ unabhängig voneinander für C₁- bis C₈-Alkylen stehen,
die Reihenfolge der Alkylenoxideinheiten beliebig ist und
v und w unabhängig voneinander für eine ganze Zahl von 0 bis 200 stehen, wobei die Summe aus v und w > 0 ist,
- Polysiloxanen der allgemeinen Formel V.2 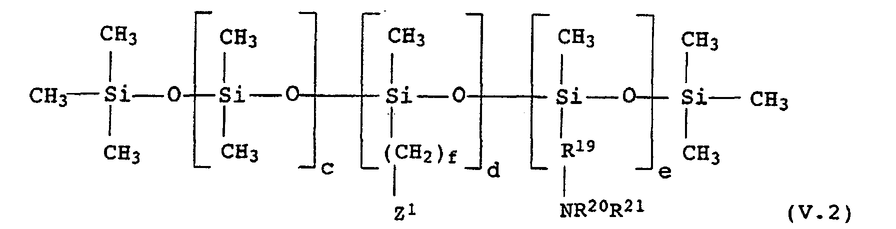 worin
R¹⁹ für einen C₁- bis C₈-Alkylenrest steht,
R²⁰ und R²¹ unabhängig voneinander für Wasserstoff, C₁bis C₈-Alkyl oder C₅- bis C₈-Cycloalkyl stehen,
die Reihenfolge der Siloxaneinheiten beliebig ist,
c, d und e unabhängig voneinander für 0 bis 100 stehen, wobei die Summe aus c, d und e mindestens 3 ist,
f für eine ganze Zahl von 2 bis 8 steht,
Z¹ für einen Rest der Formel VI
-R²²-(CH₂CH₂O)_{g}(CH₂CH(CH₃)O)ₕ-R²³ (VI)
steht, worin
die Reihenfolge der Alkylenoxideinheiten beliebig ist und g und h unabhängig voneinander für eine ganze Zahl von 0 bis 200 stehen, wobei die Summe aus g und h > O ist,
R²² für einen C₁- bis C₈-Alkylenrest steht und
R²³ für Wasserstoff oder einen C₁- bis C₈-Alkylrest steht.
und Mischungen davon.

9. Polymeres Salz nach einem der vorhergehenden Ansprüche, wobei die Komponente VA) wenigstens ein pyrrolidongruppenhaltiges Polymer, wie in Anspruch 4 definiert, mit mindestens zwei freien primären, sekundären und/oder tertiären Aminogruppen pro Molekül umfasst, oder wobei die Komponente VB) wenigstens ein pyrrolidongruppenhaltiges Polymer, wie in Anspruch 4 definiert, mit mindestens zwei freien Carbonsäuregruppen pro Molekül umfasst.

10. Polymeres Salz nach einem der vorhergehenden Ansprüche, wobei das Verhältnis von
A) freien Säuregruppen des Polymers PA) zu freien Aminogruppen der Verbindung VA), oder
B) freien Aminogruppen des Polymers PB) zu freien Säuregruppen der Verbindung VB)
in einem Bereich von 1:0,005 bis 1:1, bevorzugt 1:0,01 bis 1:0,5, liegt.

11. Kosmetisches oder pharmazeutisches Mittel, das wenigstens ein polymeres Salz nach einem der Ansprüche 1 bis 10 enthält.

12. Kosmetisches Mittel nach Anspruch 11 in Form eines Haarbehandlunqsmittels, enthaltend
a) 0,5 bis 20 Gew.-% mindestens eines polymeren Salzes, wie in einem der Ansprüche 1 bis 10 definiert,
b) 50 bis 99,5 Gew.-% wenigstens eines Lösungsmittels, ausgewählt unter Wasser, wassermischbaren Lösungsmitteln und Mischungen davon,
c) 0 bis 70 Gew.-%, bevorzugt 1 bis 50 Gew.-%, eines Treibmittels,
d) 0 bis 10 Gew.-% eines von a) verschiedenen, in Wasser löslichen oder dispergierbaren Haarpolymers,
e) 0 bis 0,5 Gew.-% einer wasserlöslichen oder wasserdispergierbaren Siliconverbindung.

13. Verwendung der polymeren Salze, wie in einem der Ansprüche 1 bis 10 definiert, als Hilfsmittel in der Kosmetik, bevorzugt als Beschichtungsmittel für Haar, Haut und Nägel, insbesondere in der Haarkosmetik, vorzugsweise als Festigerpolymer in Haarsprays, Schaumfestigern, Haarmousse, Haargel und Shampoos, in der dekorativen Kosmetik, bevorzugt in Mascara und Lidschatten, sowie als Hilfsmittel in der Pharmazie, bevorzugt als oder in Beschichtungsmittel(n) oder Bindemittel(n) für feste Arzneiformen.

14. Verwendung der polymeren Salze, wie in einem der Ansprüche 1 bis 10 definiert, als oder in Beschichtungsmittel(n) für die Textil-, Papier-, Druck-, Leder- und Klebstoffindustrie.

15. Verwendung der pyrrolidongruppenhaltigen polymeren Salze, wie in den Ansprüchen 4 und 9 definiert, in der Hautkosmetik, bevorzugt in Cremes.

## Claims

1. A water-soluble or water-dispersible polymeric salt of:
A) at least one polymer PA) having free acid groups and a neutralizing agent which comprises at least one compound VA) having at least two free amino groups per molecule, or
B) at least one polymer PB) having free amino groups and a neutralizing agent which comprises at least one di- or polyvalent inorganic acid and/or at least one compound VB) having at least two free acid groups per molecule,
where the compounds VA) and VB) additionally have at least one hydrophilic group, which is chosen from other ionogenic and/or ionic groups, divalent radicals of polyethers, divalent radicals of pyrrolidone-containing polymers and combinations thereof.

2. A polymeric salt as claimed in claim 1, where the component PA) or PB) comprises at least one polyurethane which comprises, in incorporated form,
a) at least one polymer having at least two active hydrogen atoms per molecule,
b) at least one compound which has two active hydrogen atoms and at least one ionogenic and/or ionic group per molecule,
c) optionally at least one compound having a molecular weight in the range from 56 to 500, which contains two active hydrogen atoms per molecule, and
d) at least one diisocyanate.

3. A polymeric salt as claimed in either of claims 1 or 2, where the component PA) comprises at least one polymer which comprises, in copolymerized form,
e) at least one α,β-ethylenically unsaturated mono- and/or dicarboxylic acid,
f) at least one α,β-ethylenically unsaturated monomer, which is chosen from esters of α,β-ethylenically unsaturated mono- and/or dicarboxylic acids with straight-chain and/or branched C₁-C₆-alkanols, amides of α,β-ethylenically unsaturated mono- and/or dicarboxylic acids with mono- and dialkylamines having straight-chain and/or branched C₁-C₆-alkyl radicals and mixtures thereof,
g) optionally at least one other monomer, different from e) and f), having at least one α,β-ethylenically unsaturated double bond.

4. A polymeric salt as claimed in any of the preceding claims,
where the component PA) or PB) comprises at least one pyrrolidone-containing polymer obtainable by reacting a monomeric mixture which comprises
h) itaconic acid and/or a derivative thereof, and
i) at least one diamine of the formula I
H₂N-A-NHR¹ (I)
in which
R¹ is hydrogen or C₁-C₄-alkyl,
A is a C₂-C₂₀-alkylene radical, which can be interrupted by at least one or more nonadjacent, identical or different -NR²- groups, where R² is hydrogen, C₁-C₄-alkyl, C₅-C₈-cycloalkyl, phenyl or phenyl-C₁-C₄-alkyl.

5. A polymeric salt as claimed in any of the preceding claims, where the component VA) comprises at least one polyamine of the formula II 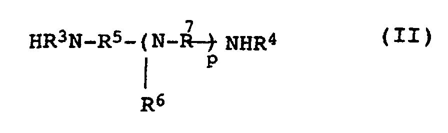 in which
p is an integer from 0 to 4,
R³ and R⁴ independently of one another are hydrogen, C₁- to C₄₀-alkyl or C₆- to C₄₀-alkenyl, where the alkyl and alkenyl radicals can carry at least one ionogenic and/or ionic group which is chosen from -COOY, -SO₃Y and -PO₃Y, where Y is H, Li, Na, K or ammonium, where, if p = 0, at least one of the radicals R³ or R⁴ is a C₁- to C₄₀-alkyl or C₆- to C₄₀-alkenyl radical which carries at least one ionogenic and/or ionic group,
R⁵ and R⁷ are a C₂- to C₆-alkylene radical, where, if p is > 1, the radicals R⁷ are chosen independently from C₂- to C₆-alkylene radicals,
R⁶ is C₁- to C₆-alkyl, C₅- to C₈-cycloalkyl, phenyl or phenyl-C₁-C₄-alkyl, where, if p is > 1, the radicals R⁶ are chosen independently from these meanings.

6. A polymeric salt as claimed in any of the preceding claims, where the component VA) comprises at least one diaminopolyether of the formula III
HR⁸N-R¹⁰-O-(CH₂CH₂O)_{q}(C₃H₆O)ᵣ-R¹¹-NHR⁹ (III)
in which
R⁸ and R⁹ independently of one another are hydrogen, C₁- to C₄₀-alkyl or C₆- to C₄₀-alkenyl, where the alkyl and alkenyl radicals may or may not carry at least one ionogenic and/or ionic group, which is chosen from -COOY, -SO₃Y and -PO₃Y, where Y is H, Li, Na, K or ammonium,
R¹⁰ and R¹¹ independently of one another are a C₂- to C₆-alkylene radical,
the order of the alkylene oxide units is arbitrary, and q and r independently of one another are an integer from 0 to 100, where the sum q + r is in a range from 5 to 100.

7. A polymeric salt as claimed in any of the preceding claims, where the component VA) comprises at least one alkoxylated amine of the formula IV
R¹²R¹⁴N-R¹⁶-NR¹³R¹⁵ (IV)
in which
R¹² and R¹³ independently of one another are C₁- to C₄₀-alkyl, C₆- to C₄₀-alkenyl, hydroxyalkyl or a radical of the formula -(CH₂CH₂O)ₘ(C₃H₆O)ₙ-H, where the order of the alkylene oxide units is arbitrary, and m and n independently of one another are an integer from 0 to 30, where the sum m + n is in a range from 1 to 30,
R¹⁴ and R¹⁵ independently of one another are chosen from the meanings given for R¹² and R¹³ and C₂- to C₆-alkyl radicals having an ionogenic or ionic group, which is chosen from -COOY, -SO₃Y and -PO₃Y, where Y is H, Li, Na, K or ammonium, and
R¹⁶ is a C₂- to C₆-alkylene radical.

8. A polymeric salt as claimed in any of the preceding claims, where the component VA) comprises at least one diaminopolyether siloxane of the formula V, which is chosen from
- polysiloxanes having repeat units of the formula V.1 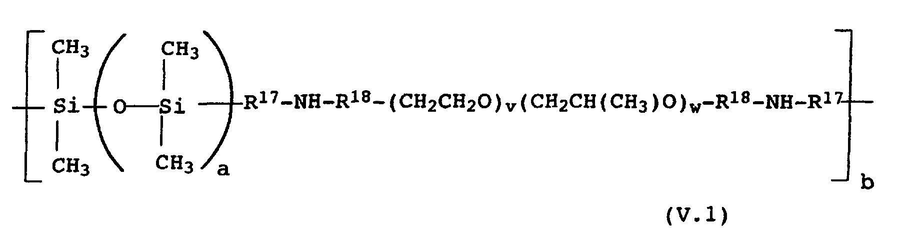 in which
a is an integer from 0 to 100,
b is an integer from 1 to 8,
R¹⁷ and R¹⁸ independently of one another are C₁- to C₈-alkylene,
the order of the alkylene oxide units is arbitrary and v and w independently of one another are an integer from
0 to 200, where the sum of v + w is > 0,
- polysiloxanes of the formula V.2 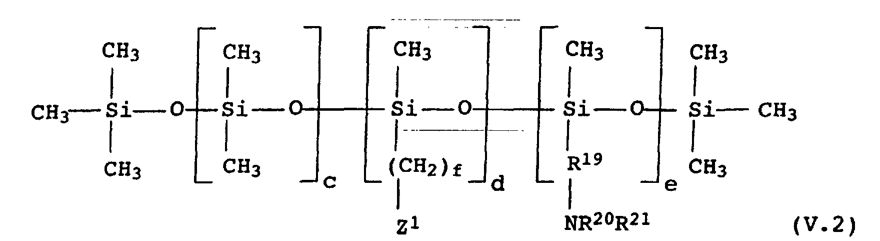 in which
R¹⁹ is a C₁- to C₈-alkylene radical,
R²⁰ and R²¹ independently of one another are hydrogen, C₁-to C₈-alkyl or C₅- to C₈-cycloalkyl,
the order of the siloxane units is arbitrary,
c, d and e independently of one another are 0 to 100,
where the sum c + d + e is at least 3,
f is an integer from 2 to 8,
z¹ is a radical of the formula VI
-R²²-(CH₂CH₂O)_{g}(CH₂CH(CH₃)O)ₕ-R²³ (VI)
in which
the order of the alkylene oxide units is arbitrary and g and h independently of one another are an integer from 0 to 200, where the sum g + h is > 0,
R²² is a C₁- to C₈-alkylene radical, and
R²³ is hydrogen or a C₁- to C₈-alkyl radical,
and mixtures thereof.

9. A polymeric salt as claimed in any of the preceding claims, where the component VA) comprises at least one pyrrolidone-containing polymer, as defined in claim 4, having at least two free primary, secondary and/or tertiary amino groups per molecule, or where the component VB) comprises at least one pyrrolidone-containing polymer, as defined in claim 4, having at least two free carboxylic acid groups per molecule.

10. A polymeric salt as claimed in any of the preceding claims,
where the ratio of
A) free acid groups in the polymer PA) to free amino groups in the compound VA), or
B) free amino groups in the polymer PB) to free acid groups in the compound VB)
is in a range from 1:0.005 to 1:1, preferably 1:0.01 to 1:0.5.

11. A cosmetic or pharmaceutical composition which comprises at least one polymeric salt as claimed in any of claims 1 to 10.

12. A cosmetic composition as claimed in claim 11 in the form of a hair-treatment composition, comprising
a) from 0.5 to 20% by weight of at least one polymeric salt, as defined in any of claims 1 to 10,
b) from 50 to 99.5% by weight of at least one solvent, chosen from water, water-miscible solvents and mixtures thereof,
c) from 0 to 70% by weight, preferably from 1 to 50% by weight, of a propellant,
d) from 0 to 10% by weight of a water-soluble or -dispersible hair polymer different from a),
e) from 0 to 0.5% by weight of a water-soluble or water-dispersible silicone compound.

13. The use of the polymeric salts, as defined in any of claims 1 to 10, as auxiliaries in cosmetics, preferably as coatings for hair, skin and nails, in particular in hair cosmetics, preferably as setting polymers in hairsprays, setting foams, hair mousse, hair gel and shampoos, in decorative cosmetics, preferably in mascara and eyeshadows, and as auxiliaries in pharmacy, preferably as or in coatings or binders for solid medicaments.

14. The use of the polymeric salts, as defined in any of claims 1 to 10, as or in coatings for the textile, paper, printing, leather and adhesives industries.

15. The use of the pyrrolidone-containing polymeric salts, as defined in claims 4 and 9, in skin cosmetics, preferably in creams.

## Revendications

1. Sel polymère soluble dans l'eau ou dispersable dans l'eau, comportant:
A) au moins un polymère PA) ayant des groupes acide libres et un agent de neutralisation, qui comporte au moins un composé VA) ayant au moins deux groupes amino libres par molécule, ou
B) au moins un polymère PB) ayant des groupes amino libres et un agent de neutralisation, qui comporte au moins un acide inorganique di- ou plurivalent et/ou au moins un composé VB) ayant au moins deux groupes acide libres par molécule,
tandis que les composés VA) et VB) présentent en outre au moins un groupe hydrophile, qui est choisi par d'autres groupes ionogènes et/ou ioniques, des restes divalents de polyéthers, des restes divalents de polymères contenant des groupes pyrrolidone, et des combinaisons de ceux-ci.

2. Sel polymère selon la revendication 1, dans lequel le composant PA) ou PB) comporte au moins un polyuréthanne, qui contient incorporés en lui
a) au moins un produit de polymérisation ayant au moins deux atomes d'hydrogène actif par molécule;
b) au moins un composé qui présente deux atomes d'hydrogène actif et au moins un groupe ionogène et/ou ionique par molécule,
c) éventuellement au moins un composé ayant une masse moléculaire dans l'intervalle de 56 à 500, qui contient deux atomes d'hydrogène actif par molécule, et
d) au moins un diisocyanate.

3. Sel polymère selon l'une des revendications 1 ou 2,
dans lequel le composant PA) comprend au moins un polymère qui contient à l'état copolymérisé
e) au moins un acide mono- et/ou dicarboxylique α,β-éthyléniquement insaturé,
f) au moins un monomère α,β-éthyléniquement insaturé, qui est choisi parmi les esters d'acides mono- et/ou dicarboxyliques α,β-éthyléniquement insaturés avec des alcanols en C₁ à C₆ linéaires et/ou ramifiés, les amides d'acides mono- et/ou dicarboxyliques α,β-éthyléniquement insaturés avec des mono- et dialkylamines ayant des restes alkyle en C₁ à C₆ linéaires et/ou ramifiés, et leurs mélanges,
g) éventuellement au moins un autre monomère, différent de e) et f), ayant au moins une double liaison α,β-éthyléniquement insaturée.

4. Sel polymère selon l'une des revendications précédentes, dans lequel le composant PA) ou PB) comporte au moins un polymère contenant des groupes pyrrolidone, pouvant être obtenu par réaction d'un mélange de monomères qui contient
h) de l'acide itaconique et/ou un dérivé de celui-ci, et
i) au moins une diamine de formule générale
**H**_{**2**}**N-A-NHR**^{**1**} (I)
où
R¹ désigne l'hydrogène ou un alkyle en C₁ à C₄,
A représente un reste alkylène en C₂ à C₂₀, qui peut être interrompu par au moins un ou plusieurs groupes -NR²- identiques ou différents, non vicinaux, tandis que R² désigne l'hydrogène, un alkyle en C₁ à C₄, un cycloalkyle en C₅ à C₈, un phényle ou un phényl-alkyle(C₁-C₄).

5. Sel polymère selon l'une des revendications précédentes, dans lequel le composant VA) comporte au moins une polyamine de formule II 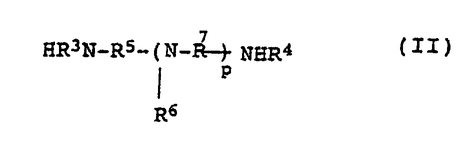 où
p représente un nombre entier de 0 à 4,
R³ et R⁴ désignent, indépendamment l'un de l'autre, l'hydrogène un alkyle en C₁ à C₄₀ ou un alcényle en C₆ à C₄₀, tandis que les restes alkyle et alcényle peuvent porter au moins un groupe ionogène et/ou ionique, qui est choisi parmi -COOY, -SO₃Y et -PO₃Y, tandis que Y représente H, Li, Na, K ou l'ammonium, tandis que pour p-=-0 au moins l'un des restes R³ ou R⁴ représente un reste alkyle en C₁ à C₄₀ ou un reste alcényle en C₆ à C₄₀, qui porte au moins un groupe ionogène et/ou ionique,
R⁵ et R⁷ désignent un reste alkylène en C₂ à C₆, tandis que pour p > 1 les restes R⁷ sont choisis indépendamment l'un de l'autre parmi les restes alkylène en C₂ à C₆,
R⁶ désigne un alkyle en C₁ à C₆, un cycloalkyle en C₅ à C₈, un phényle ou un phényl-alkyle(C₁-C₄), tandis que pour p > 1 les restes R⁶ sont choisis indépendamment l'un de l'autre parmi ces significations.

6. Sel polymère selon l'une des revendications précédentes, dans lequel le composant VA) comporte au moins un diaminopolyéther de formule III
**HR**^{**8**}**N-R**^{**10**}**-O-(CH**_{**2**}**CH**_{**2**}**O)**_{**q**}**(C**_{**3**}**H**_{**6**}**O)**_{**r**}**-R**^{**11**}**-NHR**^{**9**} (III)
où
R⁸ et R⁹ désignent indépendamment l'un de l'autre l'hydrogène, un alkyle en C₁ à C₄₀ ou un alcényle en C₆ à C₄₀, tandis que les restes alkyle et alcényle peuvent éventuellement porter au moins un groupe ionogène et/ou ionique, qui est choisi parmi -COOY, -SO₃Y et - PO₃Y, où Y représente H, Li, Na, K ou l'ammonium,
R¹⁰ et R¹¹ représentent indépendamment l'un de l'autre un reste alkylène en C₂ à C₆,
l'ordre des unités oxyde d'alkylène est quelconque et q et r désignent, indépendamment l'un de l'autre, un nombre entier de 0 à 100, tandis que la somme de q et r se situe dans l'intervalle de 5 à 100.

7. Sel polymère selon l'une des revendications précédentes, dans lequel le composant VA) comporte au moins une amine alcoxylée de formule IV
**R**^{**12**}**R**^{**14**}**N-R**^{**16**}**-NR**^{**13**}**R**^{**15**} (IV)
où
R¹² et R¹³ désignent indépendamment l'un de l'autre un alkyle en C₁ à C₄₀, un alcényle en C₆ à C₄₀, un hydroxyalkyle ou un reste de formule-(CH₂CH₂O)ₘ(C₃H₆O)ₙ-H, tandis que l'ordre des unités oxyde d'alkylène est quelconque et que m et n sont indépendamment l'un de l'autre un nombre entier de 0 à 30, tandis que la somme de m et n se situe dans l'intervalle de 1 à 30,
R¹⁴ et R¹⁵ sont choisis indépendamment l'un de l'autre parmi les significations indiquées pour R¹² et R¹³, et les restes alkyle en C₂ à C₆ ayant un groupe ionogène ou ionique qui est choisi parmi -COOY, -SO₃Y et -PO₃Y, où Y représente H, Li, Na, K ou l'ammonium, et
R¹⁶ représente un reste alkylène en C₂ à C₆.

8. Sel polymère selon l'une des revendications précédentes, dans lequel le composant VA) comporte au moins un diaminopolyéthersiloxane de formule V, qui est choisi parmi
- les polysiloxanes ayant des unités répétitives de formule générale V.1 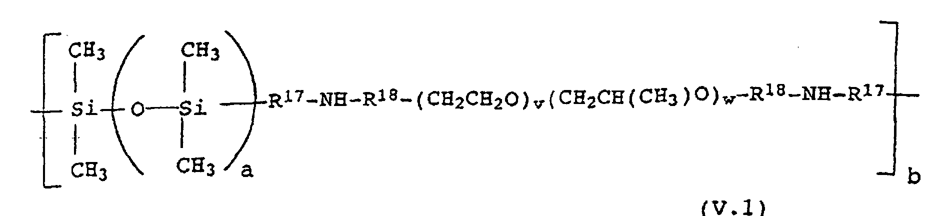
où
a désigne un nombre entier de 0 à 100,
b désigne un nombre entier de 1 à 8,
R¹⁷ et R¹⁸ représentent indépendamment l'un de l'autre un alkylène en C₁ à C₈,
l'ordre des unités oxyde d'alkylène est quelconque et
v et w sont indépendamment l'un de l'autre un nombre entier de 0 à 200, tandis que la somme de v et w est > 0,
- les polysiloxanes de formule V.2 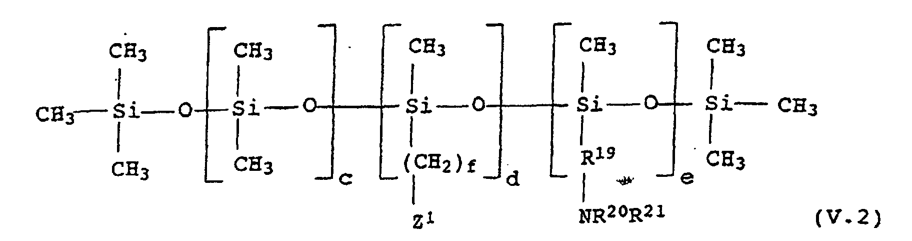
où
R¹⁹ désigne un reste alkylène en C₁ à C₈,
R²⁰ et R²¹ représentent indépendamment l'un de l'autre l'hydrogène, un alkyle en C₁ à C₈ ou un cycloalkyle en C₅ à C₈,
l'ordre des unités siloxane est quelconque,
c, d et e valent indépendamment l'un de l'autre 0 à 100, tandis que la somme de c, d et e est au moins 3,
f désigne un nombre entier de 2 à 8,
Z¹ représente un reste de formule VI
**-R**^{**22**}**-(CH**_{**2**}**CH**_{**2**}**O)**_{**g**}**(CH**_{**2**}**CH(CH**_{**3**}**)O)**_{**h**}**-R**^{**23**} (VI)
où
l'ordre des unités oxyde d'alkylène est quelconque, et
g et h désignent indépendamment l'un de l'autre un nombre entier de 0 à 200, tandis que la somme de g et h est > 0,
R²² représente un reste alkylène en C₁ à C₈ et
R²³ représente l'hydrogène ou un reste alkyle en C₁ à C₈,
et leurs mélanges.

9. Sel polymère selon l'une des revendications précédentes, dans lequel le composant VA) comporte au moins un polymère contenant des groupes pyrrolidone comme défini dans la revendication 4, avec au moins deux groupes amino libres primaires, secondaires et/ou tertiaires par molécule, ou tandis que le composant VB) comporte au moins un polymère contenant des groupes pyrrolidone comme défini dans la revendication 4, avec au moins deux groupes acide carboxylique libre par molécule.

10. Sel polymère selon l'une des revendications précédentes, dans lequel le rapport
A) des groupes acide libre du polymère PA) aux groupes amino libres du composé VA), ou
B) des groupes amino libre du polymère PB) aux groupes acide libre du composé VB)
se situe dans l'intervalle de 1:0,005 à 1:1, de préférence de 1:0,01 à 1:0,5.

11. Produit cosmétique ou pharmaceutique, qui contient au moins un sel polymère selon l'une des revendications 1 à 10.

12. Produit cosmétique selon la revendication 11 sous la forme d'un produit de traitement capillaire, contenant
a) 0,5 à 20% en poids d'au moins un sel polymère tel que défini dans l'une des revendications 1 à 10,
b) 50 à 99,5% en poids d'au moins un solvant, choisi parmi l'eau, les solvants miscibles avec l'eau et leurs mélanges,
c) 0 à 70% en poids, de préférence 1 à 50% en poids, d'un agent propulseur,
d) 0 à 10% en poids d'un polymère capillaire soluble ou dispersable dans l'eau, différent de a),
e) 0 à 0,5% en poids d'un composé de silicone hydrosoluble ou hydrodispersable.

13. Utilisation des sels polymères tels que définis dans l'une des revendications 1 à 10, comme adjuvants en cosmétique, de préférence comme agent d'enrobage pour cheveux, peau et ongles, en particulier en cosmétique capillaire, de préférence comme polymère de renforcement dans des sprays capillaires, des renforçateurs de mousse, des gels capillaires, des mousses capillaires et des shampooings, en cosmétique décorative, de préférence dans le mascara et dans les ombres à paupières, ainsi que comme adjuvants en pharmacie, de préférence comme ou dans un ou des agents de revêtement ou un ou des agents de liaison pour des formes galéniques solides.

14. Utilisation des sels polymères tels que définis dans l'une des revendications 1 à 10, comme ou dans un ou des agents de revêtement pour l'industrie textile, l'industrie papetière, l'imprimerie, l'industrie du cuir et l'industrie des colles et adhésifs.

15. Utilisation des sels polymères contenant de la pyrrolidone tels que définis dans les revendications 4 et 9, en cosmétique pour la peau, en particulier dans des crèmes.
